# EUROPÄISCHE PATENTANMELDUNG

(11) **EP 4 850 736 A2**
(43) Veröffentlichungstag der Anmeldung: **09.12.2026**
(21) Anmeldenummer: 26212732.7
(22) Anmeldetag: 02.03.2022
(51) Int. Cl.: A61M 16/20

(54) **PHYSIOLOGISCHER SIMULATOR EINER LUNGE**

(30) Priorität: 10.03.2021 DE 102021001279
(62) Dokumentnummer(n) der früheren Anmeldung(en) nach Art. 76 EPÜ: 22159794.1 / 4 057 261
(71) Anmelder: Löwenstein Medical Technology S.A., 2557 Luxembourg (LU)
(72) Erfinder: Pattai, Steffen, 56068 Koblenz (DE); Dombrowa, Mikaela Maria, 71263 Weil der Stadt (DE); Best, Alexander, 56377 Seelbach (DE)
(74) Vertreter: Löwenstein Medical IP

(57) **Zusammenfassung**

Die Erfindung betrifft einen Lungensimulator (1) zur teilweisen Simulation von Funktionen einer Lunge, umfassend zumindest einen Gaskreislauf (1a), wobei der Gaskreislauf (1a) mit einem Beatmungsgerät (2) verbunden ist, wobei das Beatmungsgerät (2) dazu eingerichtet ist, zumindest zeitweise ein Atemgas in und/oder aus den Gaskreislauf (1a) zu fördern, wobei der Lungensimulator (1) zumindest eine Vorrichtung (11) zur Einstellung der O2-Konzentration des Atemgases in dem Gaskreislauf (1a), zumindest eine Vorrichtung (12) zur Einstellung der CO2-Konzentration des Atemgases in dem Gaskreislauf (1a) und zumindest eine Vorrichtung (13) zur Simulation der mechanischen Lungenbewegung umfasst.

## Beschreibung

Die Erfindung betrifft einen Lungensimulator, welcher verschiedene Funktionen einer Lunge simulieren kann.

Bevor ein Beatmungsgerät zugelassen werden darf, ist es häufig notwendig, die Funktionen des Gerätes zu testen, um zum Beispiel eine Gefährdung des Anwenders beziehungsweise Patienten auszuschließen. Dafür bedarf es Prüfmittel, die die Atmung eines Menschen möglichst ähnlich darstellen.

Häufig werden hierzu Tiertests, beispielsweise an Schweinen, durchgeführt, um die Funktion eines Beatmungsgerätes, insbesondere bei Anästhesiegeräten, zu testen. Aus dem Stand der Technik sind zudem beispielsweise Testlungen bekannt, welche zumeist darauf beschränkt sind, die rein mechanische Funktion der Lunge wiederzugeben, also das Strömen von Atemgas in und aus der Lunge.

Aufgabe der vorliegenden Erfindung ist daher eine Vorrichtung bereitzustellen, welche ein zuverlässiges Testen von Beatmungsgeräten ermöglicht. Die Aufgabe wird durch den Lungensimulator gemäß Anspruch 1 gelöst.

Beansprucht wird ein Lungensimulator zur teilweisen Simulation von Funktionen einer Lunge, umfassend zumindest einen Gaskreislauf, wobei der Gaskreislauf mit einem Beatmungsgerät verbunden ist, wobei das Beatmungsgerät dazu eingerichtet ist, zumindest zeitweise ein Atemgas in und/oder aus den Gaskreislauf zu fördern, wobei der Lungensimulator zumindest eine Vorrichtung zur Einstellung der O2-Konzentration des Atemgases in dem Gaskreislauf, zumindest eine Vorrichtung zur Einstellung der CO2-Konzentration des Atemgases in dem Gaskreislauf und zumindest eine Vorrichtung zur Simulation der mechanischen Lungenbewegung umfasst. Das Beatmungsgerät kann dabei eine Vorrichtung zur maschinellen und/oder manuellen Beatmung, etwa ein Beatmungsbeutel, sein. Es kann alternativ oder ergänzend vorgesehen sein, dass der Lungensimulator ohne Beatmungsgerät betrieben wird, wobei die Förderung des Atemgases im Gaskreislauf und/oder in/aus dem Gaskreislauf durch die Vorrichtung zur Simulation der mechanischen Lungenbewegung durchgeführt wird.

In manchen Ausführungsformen kennzeichnet den Lungensimulator, dass der Lungensimulator einen Spülgaslauf umfasst, durch welchen ein Spülgas strömt.

In manchen Ausführungsformen kennzeichnet den Lungensimulator, dass die Vorrichtung zur Einstellung der O2-Konzentration ein Gastauscher ist.

In manchen Ausführungsformen kennzeichnet den Lungensimulator, dass der Gastauscher dazu eingerichtet ist über das Spülgas des Spülgaslaufs die O2-Konzentration des Atemgases in dem Gaskreislauf einzustellen.

In manchen Ausführungsformen kennzeichnet den Lungensimulator, dass in dem Gastauscher zumindest stellenweise und/oder zeitweise O2-Moleküle und/oder CO2-Moleküle und/oder N2-Moleküle vom Atemgas in das Spülgas übergehen und/oder vom Spülgas in das Atemgas übergehen.

In manchen Ausführungsformen kennzeichnet den Lungensimulator, dass der Gastauscher zumindest eine Membran umfasst.

In manchen Ausführungsformen kennzeichnet den Lungensimulator, dass die Membran zumindest für CO2 und/oder O2 und/oder N2 permeabel ist.

In manchen Ausführungsformen kennzeichnet den Lungensimulator, dass die Membran so eingerichtet und ausgebildet ist, dass entlang der Membran zumindest O2-Moleküle und/oder CO2-Moleküle und/oder N2-Moleküle vom Atemgas in das Spülgas übergehen und/oder vom Spülgas in das Atemgas übergehen.

In manchen Ausführungsformen kennzeichnet den Lungensimulator, dass der Gastauscher zumindest zwei Gasräume umfasst, wobei die beiden Gasräume zumindest durch die Membran voneinander getrennt sind.

In manchen Ausführungsformen kennzeichnet den Lungensimulator, dass der Gastauscher so eingerichtet und ausgebildet ist, dass ein Atemgas auf der einen Seite an der mindestens einen Membran und ein Spülgas auf der anderen Seite der mindestens einen Membran zumindest stellenweise entlang strömt.

In manchen Ausführungsformen kennzeichnet den Lungensimulator, dass die zumindest eine Membran als zumindest eine Hohlfaser ausgebildet ist, wobei die zumindest eine Hohlfaser einen der zwei Gasräume umschließt.

In manchen Ausführungsformen kennzeichnet den Lungensimulator, dass der Gastauscher zumindest vier Gasräume umfasst, wobei ein erster Gasraum durch zumindest eine Hohlfaser gasleitend mit einem zweiten Gasraum verbunden ist.

In manchen Ausführungsformen kennzeichnet den Lungensimulator, dass die zumindest eine Hohlfaser durch den dritten Gasraum läuft.

In manchen Ausführungsformen kennzeichnet den Lungensimulator, dass die zumindest eine Hohlfaser eine Polymethylpenten-Hohlfaser oder eine Polypropylen-Hohlfaser ist.

In manchen Ausführungsformen kennzeichnet den Lungensimulator, dass am ersten Gasraum ein Anschluss angeordnet ist, über den das Spülgas in den ersten Gasraum geleitet wird, und wobei das Spülgas über die zumindest eine Hohlfaser vom ersten Gasraum in den zweiten Gasraum geleitet wird, wobei am zweiten Gasraum ein Anschluss angeordnet ist, über welchen das Spülgas aus dem zweiten Gasraum geleitet wird.

In manchen Ausführungsformen kennzeichnet den Lungensimulator, dass an dem dritten Gasraum zumindest zwei Anschlüsse angeordnet sind, über welche das Atemgas in den dritten Gasraum hinein und/oder hinausgeleitet wird.

In manchen Ausführungsformen kennzeichnet den Lungensimulator, dass die Anschlüsse des dritten Gasraumes so eingerichtet und angeordnet sind, dass das Atemgas den dritten Gasraum zumindest teilweise durchströmt und dabei die zumindest eine Hohlfaser zumindest teilweise umströmt.

In manchen Ausführungsformen kennzeichnet den Lungensimulator, dass die zumindest eine Hohlfaser so eingerichtet und ausgebildet ist, dass zumindest teilweise ein Gasaustausch zwischen dem Spülgas in der Hohlfaser und dem die Hohlfaser umströmenden Atemgas stattfindet.

In manchen Ausführungsformen kennzeichnet den Lungensimulator, dass das Spülgas eine geringere O2-Konzentration als das Atemgas aufweist und in dem Gastauscher O2-Moleküle aus dem Atemgas in das Spülgas übergehen.

In manchen Ausführungsformen kennzeichnet den Lungensimulator, dass der Sauerstofftauscher ein Oxygenator ist, wobei das Atemgas des Gaskreislaufs durch den Blutweg geleitet wird und der Spülgaslauf durch den Atemgasweg geleitet wird.

In manchen Ausführungsformen kennzeichnet den Lungensimulator, dass der Spülgaslauf zumindest eine Gasquelle umfasst, aus welcher ein Gas in den Spülgaslauf geleitet wird.

In manchen Ausführungsformen kennzeichnet den Lungensimulator, dass das Spülgas aus zumindest zwei Gasen besteht, welche aus zumindest zwei Gasquellen in den Spülgaslauf geleitet werden und in einem Gasmischer gemischt werden.

In manchen Ausführungsformen kennzeichnet den Lungensimulator, dass eine der zumindest zwei Gasquellen eine Sauerstoffquelle und/oder eine CO2-Quelle ist.

In manchen Ausführungsformen kennzeichnet den Lungensimulator, dass aus einer der zumindest zwei Gasquellen Luft und/oder Stickstoff in den Spülgaslauf geleitet wird.

In manchen Ausführungsformen kennzeichnet den Lungensimulator, dass der Gasmischer zumindest ein Ventil umfasst.

In manchen Ausführungsformen kennzeichnet den Lungensimulator, dass der Gasmischer zumindest eine Vorrichtung zur Messung eines Differenzdruckes umfasst.

In manchen Ausführungsformen kennzeichnet den Lungensimulator, dass der Gasmischer zumindest eine Mischkammer umfasst.

In manchen Ausführungsformen kennzeichnet den Lungensimulator, dass das zumindest eine Ventil des Gasmischers ein Proportionalventil ist.

In manchen Ausführungsformen kennzeichnet den Lungensimulator, dass im Spülgaslauf zumindest ein Sauerstoffsensor angeordnet.

In manchen Ausführungsformen kennzeichnet den Lungensimulator, dass im Spülgaslauf zumindest ein Ventil angeordnet ist.

In manchen Ausführungsformen kennzeichnet den Lungensimulator, dass das zumindest eine Ventil ein 3/2-Wege-Ventil ist.

In manchen Ausführungsformen kennzeichnet den Lungensimulator, dass das Ventil zwischen einem ersten Sauerstoffsensor, dem Gastauscher und einem zweiten Sauerstoffsensor angeordnet ist, wobei der zweite Sauerstoffsensor mit dem Ventil und dem Gastauscher gasleitend verbunden ist.

In manchen Ausführungsformen kennzeichnet den Lungensimulator, dass in dem Spülgaslauf zumindest ein Differenzdruckmesser, angeordnet ist.

In manchen Ausführungsformen kennzeichnet den Lungensimulator, dass der Druckmesser in Flussrichtung nach dem Gastauscher angeordnet ist.

In manchen Ausführungsformen kennzeichnet den Lungensimulator, dass die Vorrichtung zur Einstellung der CO2-Konzentration des Atemgases in dem Gaskreislauf eine CO2-Beimischer ist.

In manchen Ausführungsformen kennzeichnet den Lungensimulator, dass die Vorrichtung zur Einstellung der CO2-Konzentration in Flussrichtung im Gaskreislauf nach der Vorrichtung zur Einstellung der O2-Konzentration angeordnet ist.

In manchen Ausführungsformen kennzeichnet den Lungensimulator, dass der CO2-Beimischer mit einer CO2-Quelle verbunden ist.

In manchen Ausführungsformen kennzeichnet den Lungensimulator, dass über den CO2-Beimischer CO2 in das Atemgas in dem Gaskreislauf eingeleitet wird.

In manchen Ausführungsformen kennzeichnet den Lungensimulator, dass der CO2-Beimischer zumindest ein Ventil umfasst, wobei das Ventil mit dem Gaskreislauf verbunden ist.

In manchen Ausführungsformen kennzeichnet den Lungensimulator, dass im Gaskreislauf die Vorrichtung zur Simulation der mechanischen Lungenbewegung in Flussrichtung nach der Vorrichtung zur Einstellung der CO2-Konzentration angeordnet ist.

In manchen Ausführungsformen kennzeichnet den Lungensimulator, dass die Vorrichtung zur Simulation der mechanischen Lungenbewegung eine Testlunge ist.

In manchen Ausführungsformen kennzeichnet den Lungensimulator, dass zwischen dem Gastauscher und der Testlunge die Verbindung des Beatmungsgerätes mit dem Gaskreislauf angeordnet ist.

In manchen Ausführungsformen kennzeichnet den Lungensimulator, dass in dem Gaskreislauf in Flussrichtung nach der Vorrichtung zur Einstellung der O2-Konzentration zumindest ein Ventil angeordnet ist.

In manchen Ausführungsformen kennzeichnet den Lungensimulator, dass zwischen der Vorrichtung zur Einstellung der O2-Konzentration und der Vorrichtung zur Simulation der mechanischen Lungenbewegung zumindest ein Ventil angeordnet ist und in Flussrichtung nach der Vorrichtung zur Simulation der mechanischen Lungenbewegung zumindest ein Ventil angeordnet ist.

In manchen Ausführungsformen kennzeichnet den Lungensimulator, dass das zumindest eine Ventil ein Rückschlagventil ist.

In manchen Ausführungsformen kennzeichnet den Lungensimulator, dass das zwischen dem Gastauscher und dem CO2-Beimischer ein Ventil angeordnet ist.

In manchen Ausführungsformen kennzeichnet den Lungensimulator, dass zwischen der Testlunge und der Verbindung des Beatmungsgerätes mit dem Gaskreislauf ein Ventil angeordnet ist.

In manchen Ausführungsformen kennzeichnet den Lungensimulator, dass die Ventile Rückschlagventile und/oder steuerbare Ventile sind.

In manchen Ausführungsformen kennzeichnet den Lungensimulator, dass die Vorrichtung zur Einstellung der CO2-Konzentration des Gases im Gaskreislauf ein Gastauscher ist.

In manchen Ausführungsformen kennzeichnet den Lungensimulator, dass die Vorrichtung zur Einstellung der CO2-Konzentration und die Vorrichtung zur Einstellung der O2-Konzentration in einem Gastauscher kombiniert sind.

In manchen Ausführungsformen kennzeichnet den Lungensimulator, dass das Spülgas eine höhere CO2-Konzentration als das Atemgas aufweist und in dem Gastauscher CO2-Moleküle von dem Spülgas in das Atemgas übergehen.

In manchen Ausführungsformen kennzeichnet den Lungensimulator, dass in dem Gaskreislauf zumindest ein Filter angeordnet ist.

In manchen Ausführungsformen kennzeichnet den Lungensimulator, dass der Filter dazu eingerichtet ist Gasbestandteile zumindest teilweise zu filtern.

In manchen Ausführungsformen kennzeichnet den Lungensimulator, dass der Filter dazu eingerichtet ist Anästhesiegase zumindest teilweise aus dem Gas des Gaskreislaufs zu filtern.

In manchen Ausführungsformen kennzeichnet den Lungensimulator, dass der Filter ein Aktivkohlefilter ist.

In manchen Ausführungsformen kennzeichnet den Lungensimulator, dass der Lungensimulator eine Vorrichtung zur Einstellung der Atemgasfeuchtigkeit umfasst.

In manchen Ausführungsformen kennzeichnet den Lungensimulator, dass die Vorrichtung zur Einstellung der Atemgasfeuchtigkeit ein Atemgasanfeuchter ist.

In manchen Ausführungsformen kennzeichnet den Lungensimulator, dass der Atemgasanfeuchter in Flussrichtung nach der Testlunge und vor dem Anschluss des Beatmungsgeräts in dem Gaskreislauf angeordnet ist.

In manchen Ausführungsformen kennzeichnet den Lungensimulator, dass in dem Gaskreislauf (1a) zumindest eine Vorrichtung zur Simulation von Blockaden der Atemwege und/oder Apnoen und/oder Atemaussetzern angeordnet ist.

In manchen Ausführungsformen kennzeichnet den Lungensimulator, dass zwischen dem Lungensimulator und dem Beatmungsgerät ein künstlicher Kopf mit Maske angeordnet ist und der Gaskreislauf zumindest teilweise durch den künstlichen Kopf führt und der Gaskreislauf und der Spülgaslauf in einem Gasmischer zusammentreffen, wobei der Gasmischer als Mischkammer ausgeführt ist und wobei eine Pumpe mit der Mischkammer verbunden ist, über welche zumindest eine definierte Menge an Sauerstoff aus der Mischkammer entfernt wird und wobei die entfernte Menge Sauerstoff durch eine entsprechende Menge an CO2 aus einer Gasquelle ersetzt wird.

Die Erfindung betrifft auch einen Gastauscher, beispielsweise zum Einsatz in einem Lungensimulator. Der Gastauscher umfasst zumindest zwei Gasräume, wobei die beiden Gasräume zumindest durch eine Membran voneinander getrennt sind. Den Gastauscher kennzeichnet dabei, dass der Gastauscher so eingerichtet und ausgebildet ist, dass ein Atemgas auf der einen Seite an der mindestens einen Membran und ein Spülgas auf der anderen Seite der mindestens einen Membran zumindest stellenweise entlang strömt und die zumindest eine Membran als zumindest eine Hohlfaser ausgebildet ist, wobei die zumindest eine Hohlfaser einen der zwei Gasräume umschließt und wobei die Membran zumindest zeitweise und/oder stellenweise für O2-Moleküle und/oder CO2-Moleküle und/oder N2-Moleküle permeabel ist.

In manchen Ausführungsformen kennzeichnet den Gastauscher, dass der Gastauscher zumindest vier Gasräume umfasst, wobei ein erster Gasraum durch zumindest die zumindest eine Hohlfaser gasleitend mit einem zweiten Gasraum verbunden ist und die zumindest eine Hohlfaser durch den dritten Gasraum läuft und am ersten Gasraum ein Anschluss angeordnet ist, über den das Spülgas in den ersten Gasraum geleitet wird, und wobei das Spülgas über die zumindest eine Hohlfaser vom ersten Gasraum in den zweiten Gasraum geleitet wird, wobei am zweiten Gasraum ein Anschluss angeordnet ist, über welchen das Spülgas aus dem zweiten Gasraum geleitet wird.an dem dritten Gasraum zumindest zwei Anschlüsse angeordnet sind, über welche das Atemgas in den dritten Gasraum hinein und/oder hinausgeleitet wird, wobei die Anschlüsse des dritten Gasraumes so eingerichtet und angeordnet sind, dass das Atemgas den dritten Gasraum zumindest teilweise durchströmt und dabei die zumindest eine Hohlfaser zumindest teilweise umströmt und wobei die zumindest eine Hohlfaser so eingerichtet und ausgebildet ist, dass zumindest teilweise ein Gasaustausch zwischen dem Spülgas in der Hohlfaser und dem die Hohlfaser umströmenden Atemgas stattfindet.

Das Verfahren zur teilweisen Simulation von Funktionen einer Lunge, kennzeichnet, dass in einem Lungensimulator nach zumindest einem der vorhergehenden Ansprüche die O2-Konzentration und die CO2-Konzentration eines in einem Gaskreislauf geführten Atemgases eingestellt werden und die mechanischen Lungenbewegungen simuliert werden. Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Der erfinderische Lugensimulator lässt sich beispielsweise für Übungszwecke von Ärzten, Pflegern und anderen Betreuern einsetzen. Es können etwa verschiedene Situationen nachgestellt werden, bei denen die Bedienung eines Beatmungsgerätes geübt werden kann. Es lassen sich etwa verschiedene Patientenbedingungen nachstellen, auf die der Betreuer/Arzt/Pfleger reagieren kann. Beispielsweise kann über verschiedene gezielte Einstellungen der Gasströmungen, insbesondere des Sauerstoff-, CO2- und Stickstoffflusses. Mit Hilfe des Lungensimulators können Betreuer/Ärzte/Pfleger auf realitätsnahe Situationen der Beatmung geschult werden.

Auch kann anhand des Lungensimulators die manuelle Beatmung, etwa über einen Beatmungsbeutel, geübt werden. Es kann dazu vorgesehen sein, dass in dem Gaskreislauf zusätzliche Sensoren angebracht werden, beispielsweise um die Effektivität der Beatmung zu bestimmen. Durch gezielt gewählte Ausführungen der Testlunge (als Vorrichtung zur Simulation der Lungenbewegung) können so auch verschiedene Beatmungswiderstände realisiert werden. Zusätzlich oder ergänzend können dazu auch pneumatische Widerstände in den Gaskreislauf angeordnet werden.

Neben dem Einsatz als Trainingstool können auch Neuentwicklungen am Lungensimulator getestet werden. Über die Simulation von bestimmten Situationen kann beispielsweise die Reaktion des Beatmungsgerätes überprüft werden. Auch können zum Beispiel Beatmungstests über einen längeren Zeitraum durchgeführt werden. Insbesondere Beatmungstests, bei denen derzeit Tierversuche nötig sind, können so vermieden werden.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Lebewesens (z.B. Patient und/oder Neugeborener und/oder Frühgeborene) übernimmt und/oder zur Atemtherapie dient und/oder anderweitig die Atmung des Anwenders bzw. Patienten beeinflusst. Darunter fallen zum Beispiel, aber nicht ausschließend, CPAP- sowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können. Im Sinne der Beschreibung kann unter Beatmungsgerät auch eine Vorrichtung zur manuellen Beatmung, etwa ein Beatmungsbeutel, verstanden werden.

Als Patienteninterface kann, soweit nicht ausdrücklich anders beschrieben, jegliches Peripheriegerät verstanden werden, welches zur Interaktion, insbesondere zu Therapie- oder Diagnosezwecken, der Messeinrichtung mit einem Lebewesen gedacht ist. Insbesondere kann ein Patienteninterface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben bzw. -kanülen und sogenannte Nasenbrillen können als Maske beziehungsweise Patienteninterface eingesetzt werden. In manchen Fällen kann das Patienteninterface auch ein einfaches Mundstück, beispielsweise ein Rohr, sein, durch welches das Lebewesen zumindest ausatmet und/oder einatmet.

Im Zuge der Erfindung können die Funktionen einer Lunge zumindest zum Teil auch Funktionen der oberen Atemwege, wie zum Beispiel das Anfeuchten und/oder Anwärmen des Atemgases, umfassen. Auch Probleme der oberen Atemwege, wie zum Beispiel eine Blockade, können im weitesten Sinne zu den Funktionen einer Lunge gezählt werden. Entsprechend ist in manchen Ausführungsformen der Lungensimulator dazu eingerichtet, auch Funktionen der oberen Atemwege zu simulieren.

Der Lungensimulator ist im Wesentlichen aus zumindest drei Komponenten aufgebaut:
- Einer Vorrichtung zur Einstellung der O2-Konzentration
- Einer Vorrichtung zur Einstellung der CO2-Konzentration
- Einer Vorrichtung zur Simulation der mechanischen Lungenbewegung

Diese Komponenten sollen weitestgehend die physiologischen Funktionen einer echten Lunge simulieren: die Sauerstoffaufnahme aus dem Atemgas, die CO2-Abgabge an das Atemgas sowie die Atemgasströmung durch Expansion und Kompression der Lungenflügel. Dazu wird ein Atemgas in einem Kreislauf zwischen den drei Vorrichtungen geführt, wobei der Kreislauf zusätzlich mit einem Beatmungsgerät verbunden werden kann, welches Atemgas zeitweise in und optional auch aus den Kreislauf fördern kann.

Die Einstellung der O2-Konzentration erfolgt zum Beispiel über einen Gastauscher, in welchem ein Spülgas und das Atemgas entlang einer Membran strömen und über die Membran Gas austauschen können. Die Zusammensetzung des Spülgases ist dabei ausschlaggebend für die Menge an Sauerstoff, welche aus dem Atemgas gefiltert wird. Die Membran ist beispielsweise eine Polymethylpenten-Membran. Andere Membranen sind auch denkbar, wobei viele Membranen (zum Beispiel viele Polypropylen-Membranen) eine vergleichsweise schwere Kontrolle des Sauerstoffaustauschs bieten. Für das Spülgas ist jedes Gas denkbar, wobei für eine Sauerstofffilterung aus dem Atemgas darauf zu achten ist, dass die O2-Konzentration im Spülgas unterhalb der O2-Konzentration des Atemgases liegt. Insbesondere eignen sich Luft oder Luft-ähnliche Gasgemische, wie etwa mit Sauerstoff versetzte Luft, reine Luft, mit Stickstoff verdünnte Luft, oder auch ein Gemisch aus Luft, Sauerstoff und Stickstoff. Auch eine Mischung aus Sauerstoff und Stickstoff kann als Spülgas verwendet werden.

Über ein Ventil kann zur Einstellung der CO2-Konzentration CO2 in das Atemgas geleitet werden. Dabei wird die Zugabe an CO2 auf die Sauerstofffilterung abgestimmt.

Die Simulation der mechanischen Lungenbewegung wird zum Beispiel über eine herkömmliche Testlunge realisiert. Die Testlunge kann dabei eine passive Ausführung oder auch eine mechanische Testlunge sein, welche aktiv die Lungenbewegung nachahmt. Die Richtung des Gasflusses in dem Kreislauf wird maßgeblich durch die Positionierung von Rückschlagventilen beeinflusst.

Zusätzlich können in den Lungensimulator optional noch ein Filter und/oder ein Atemgasanfeuchter angeordnet sein. Der Filter kann beispielsweise Anästhesiegase aus dem Atemgas filtern und so die Aufnahme von Anästhesiegasen durch die Lunge simulieren. Zusätzlich wird in den oberen Atemwegen das Atemgas angefeuchtet und erwärmt - diese physiologische Funktion lässt sich zum Beispiel über einen Atemgasanfeuchter simulieren.

In manchen Ausführungsformen kann der Gaskreislauf auch eine Vorrichtung enthalten, welche Atemwegsbeschwerden simulieren kann, wie zum Beispiel eine Blockade der Atemwege oder ein Aussetzen der Atmung (Apnoe). Beispielsweise kann dazu ein druckabhängiger Widerstand in dem Gaskreislauf 1a angeordnet sein, welcher sich unterhalb eines einstellbaren Atemgasdruckes nicht oder nur teilweise öffnet. Beispielsweise kann dazu auch ein druckgesteuertes Ventil verwendet werden, welches durch die Messwerte eines Drucksensors gesteuert wird, welcher unmittelbar vor dem Ventil angeordnet ist. Die Simulation von Blockaden oder Apnoen kann alternativ oder ergänzend auch durch eine (mechanische) Testlunge erreicht werden, welche über eine entsprechende Software gesteuert wird.

Dadurch, dass zumindest die Sauerstofffilterung, die CO2-Zugabe und das Atemzugsvolumen zum Teil fein einstellbar sind, kann neben der generellen Simulation einiger Funktionen der Lunge auch effektiv verschiedene Körpergrößen und gegebenenfalls Krankheiten/Symptome (wie z.B. ineffektive Sauerstoffaufnahme) simuliert werden.

Als Atemgas kann eine Variation von Gasen und Gasgemischen vom Beatmungsgerät in den Gaskreislauf gefördert werden. Ein Beispiel für Atemgas ist Luft, welche durch das Beatmungsgerät aus der Umgebung angesaugt wird und/oder als synthetische Luft und/oder Druckluft bereitgestellt wird. Zum Teil kann die Luft zusätzlich mit Sauerstoff versetzt werden um eine höhere Sauerstoffkonzentration zu erreichen. In manchen Fällen kann auch reiner Sauerstoff als Atemgas verwendet werden und/oder eine Mischung aus Stickstoff und Sauerstoff.

Als Spülgas eignet sich im Wesentlichen jedes Gas bzw. Gasgemisch, welches eine von dem Atemgas abweichende Sauerstoffkonzentration aufweist. Soll Sauerstoff aus dem Atemgas gefiltert werden, ist beispielsweise darauf zu achten, dass die Sauerstoffkonzentration des Spülgases unterhalb der Sauerstoffkonzentration des Atemgases liegt. Beispielsweise kann Luft (Umgebungsluft, Druckluft, synthetische Luft) als Spülgas verwendet werden. Auch reiner Stickstoff und/oder ein Gemisch aus Stickstoff und Sauerstoff eignet sich als Spülgas. Zudem sind auch Mischungen von Luft und Sauerstoff, Luft und Stickstoff, Luft mit Stickstoff und Sauerstoff, Luft mit CO2 und/oder Stickstoff mit Sauerstoff und CO2 als Spülgase geeignet.

Bei manchen Ausführungsformen wird der Gasaustausch in einem Gasmischer, beispielsweise einer Mischkammer, realisiert. In der Mischkammer wird die CO2-Konzentration so eingestellt, dass diese einer Umwandlung von O2 in CO2 in einer echten Lunge entspricht. Es ist beispielsweise vorgesehen, dass am Ausgang des Gaskreislaufes bzw. des Lungensimulators eine natürliche CO2-Konzentration gemessen werden kann. Dazu muss zum einen CO2 zugeführt werden und zum anderen auch Sauerstoff abgeführt werden. Über eine Pumpe wird zum Beispiel eine Menge an Atemgas abgeführt, welche eine Menge an Sauerstoff enthält, welch der natürlich umgesetzten Menge entspricht. Für einen Volumenausgleich und zur Einstellung der richtigen CO2-Konzentration wird im Gegenzug zusätzliches CO2 und N2 aus Gasquellen nachgeliefert, beispielsweise als Spülgas. Es kann dabei auch berücksichtig werden, dass bei der menschlichen Atmung etwas mehr O2 - im Bereich von 1/5 bis 1/8, beispielsweise 1/6 - aufgenommen wird als CO2 abgegeben wird. Es kann also vorgesehen sein, dass dieses Ungleichgewicht zusätzlich mit in den Gasaustausch einbezogen wird. Über eine weitere Pumpe und/oder eine Testlunge kann die Atembewegung, zumindest Inspiration und Exspiration, nachgestellt werden. Es kann ergänzend vorgesehen sein, dass in der Mischkammer Mittel zur Konvektion vorgesehen sind, beispielsweise ein Lüfter, um eine gute Durchmischung der Gase zu realisieren. Zudem sind Sauerstoff- und CO2-Sensoren vorgesehen, um die Konzentration und/oder Menge an Sauerstoff und CO2 zu messen, welche ausgetauscht wird und/oder im Atemgas vorhanden ist. Zusätzlich können Druck- und oder Flusssensoren vorgesehen sein, beispielsweise um die simulierten Atemdrücke und/oder Atemflüsse zu messen und/oder einstellen zu können. Der Gaskreislauf wird im Wesentlichen durch den Gasfluss vom Eingang des Lungensimulators, beispielsweise am Übergang vom künstlichen Kopf, durch die Mischkammer in die Pumpe/Testlunge und zurück beschrieben. Der Spülgaslauf wird hier beispielsweise durch den Gasfluss von den Gasquellen (CO2, N2) durch die Mischkammer und durch die Pumpe aus der Mischkammer bzw. dem Lungensimulator beschrieben. Spülgaslauf und Gaskreislauf (Atemgas) treffen also in der Mischkammer aufeinander.

Beispielsweise kann auch vorgesehen sein, dass zwischen dem Lungensimulator und einem angeschlossenen Beatmungsgerät ein künstlicher Kopf und/oder eine Maske angeordnet sind. Beispielsweise kann vorgesehen sein, den Lungensimulator als Prüfstand für Beatmungsmasken zu verwenden, wobei der Lungensimulator die Atmung nachstellt und verschiedene Effekte der jeweiligen Maske beobachtet bzw. gemessen werden können. Um beispielsweise den Effekt von Sauerstoffbeimischung zum Atemgas, welches beispielsweise das Beatmungsgerät fördert, zu prüfen, kann vorgesehen sein, dass immer eine genau definierte Menge an Sauerstoff aus der Mischkammer gepumpt wird. So kann im Gegenzug beispielsweise auch eine Aufkonzentrierung von CO2 durch Rückatmung geprüft werden. Durch die Entfernung von einer konstant gleichen Menge an Sauerstoff aus der Mischkammer und einer konstant gleichen Menge an CO2, welcher von den Gasquellen in die Mischkammer strömt, wird beispielsweise ein konstanter Umsatz von CO2 dargestellt. Entsprechend können die Effekte der Sauerstoff und CO2 Veränderung, etwa durch die Maske, geprüft werden.

Bei manchen Ausführungsformen kann auch vorgesehen sein, dass der Lungensimulator die Atmung vollständig simuliert und auch kein Beatmungsgerät mit dem Lungensimulator verbunden sein muss. Beispielsweise ist der Lungensimulator dabei so eingerichtet, dass, etwa über die Testlunge, die Atembewegung nachgestellt wird und so die Umgebungsluft eingesaugt wird. Beispielsweise lässt sich so der Lungensimulator zusammen mit einem künstlichen Kopf nutzen um medizinische Schutzmasken und/oder Arbeitsschutzmasken zu testen. Auch können so andere Atemeffekte geprüft werden, wie etwa die Veränderung der Gaskonzentrationen in der Umgebungsluft.

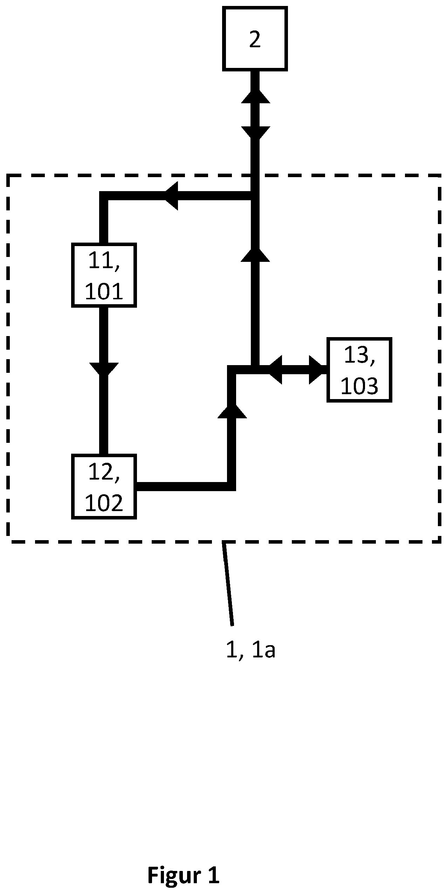

Die Erfindung wird anhand der Figuren 1 bis 8 beispielhaft näher beschrieben.

Die Pfeilrichtungen deuten in den Figuren auf die Fluss- bzw. Strömungsrichtung des jeweiligen Gases hin. Weist eine Verbindung, beispielsweise zwischen zwei Elementen, zwei Pfeilrichtungen auf, soll dargestellt, dass in beide Richtungen strömen kann. Beispielsweise unterscheidet sich die Strömungsrichtung/Flussrichtung dann je nach (simulierter) Atemphase.

Die einzelnen Vorrichtungen und Elemente innerhalb des Lungensimulators sind, soweit erforderlich, gasleitend miteinander verbunden. Die gasleitende Verbindung wird beispielsweise durch Schläuche und/oder Rohrleitungen realisiert. In manchen Ausführungsformen können die Vorrichtungen, zumindest teilweise, auch direkt aneinandergereiht werden, so dass der Ausgang einer Vorrichtung direkt mit dem Eingang der nächsten Vorrichtung verbunden wird.

Die Anordnung der Vorrichtungen und Elemente ist in den Figuren beispielhaft beschrieben. Es ist darauf hinzuweisen, dass die Vorrichtungen und Elemente im Lichte der Erfindung auch in anderer Reihenfolge angeordnet werden können.

Eine beispielhafte Ausführungsform des Lungensimulators 1 zusammen mit einem Beatmungsgerät 2 ist schematisch in Figur 1 dargestellt. Der Lungensimulator 1 umfasst beispielhaft einen Gaskreislauf 1a, welcher mit dem Beatmungsgerät 2 verbunden ist. Innerhalb des Gaskreislaufs 1a sind eine Vorrichtung 11 zur Einstellung der O2-Konzentration, eine Vorrichtung 12 zur Einstellung der CO2-Konzentration und eine Vorrichtung 13 zur Simulation mechanischen Lungenbewegung.

Das Beatmungsgerät 2 ist beispielhaft dazu ausgebildet und eingerichtet ein Gas, beispielsweise Atemgas, zu fördern. Zumindest zeitweise kann das Beatmungsgerät 2 Atemgas in den Gaskreislauf 1a fördern. Dazu verfügt das Beatmungsgerät 2 über zumindest eine Steuereinheit, welche ein Ventil einer Atemgasquelle und/oder ein Gebläse als Atemgasquelle steuern kann. Das Beatmungsgerät 2 ist zudem dazu eingerichtet, zumindest einen Atemgasdruck und/oder einen Atemgasfluss zu messen und/oder zumindest zeitweise vorzugeben. In manchen Ausführungsformen ist das Beatmungsgerät 2 dazu eingerichtet zumindest ein Druck- und oder Flussmuster vorzugeben. Beispielsweise ist das Beatmungsgerät 2 auch dazu eingerichtet Atmungsmuster und/oder Phasen der Atmung, zum Beispiel Inspiration und Exspiration, zu erkennen und/oder vorzugeben. Das Beatmungsgerät 2 ist beispielsweise dazu eingerichtet, zumindest ein erstes Druck- und/oder Flussniveau für eine Exspiration und zumindest ein zweites Druck- und/oder Flussniveau für eine Inspiration vorzugeben. Für den Lungensimulator 1 bedeutet dabei eine Inspiration, dass zumindest zeitweise Atemgas vom Beatmungsgerät 1 in den Gaskreislauf 1a gefördert wird. In einer Exspiration strömt Atemgas zumindest zeitweise aus dem Gaskreislauf 1a aus, beispielsweise in die Umgebung und/oder in das Beatmungsgerät 2. In manchen Ausführungsformen wird das Atemgas im Beatmungsgerät 2 zumindest teilweise in einem Kreislauf geführt.

In manchen Ausführungsformen verfügt das Beatmungsgerät 2 über Sauerstoff- und/oder CO2-Sensoren und/oder ist mit solchen verbunden.

In manchen Ausführungsformen ist das Beatmungsgerät 2 so eingerichtet, dass, beispielsweise über eine O2-Quelle, dem Atemgas im Beatmungsgerät 2 Sauerstoff beigemischt werden kann. In manchen Ausführungsformen erhält das Beatmungsgerät 2 über ein Pulsoxymeter Daten über die Sauerstoffsättigung des Blutes des Patienten und regelt anhand dieser Daten eine Sauerstoffzufuhr zum Atemgas. Daher wird in manchen Ausführungsformen beispielsweise eine Vorrichtung mit dem Beatmungsgerät 2 verbunden, welche Messwerte anstelle des Pulsoxymeters generiert bzw. simuliert.

In manchen Ausführungsformen regelt das Beatmungsgerät 2 eine Sauerstoffzufuhr zum Atemgas anhand der gemessenen Sauerstoffkonzentration im Atemgas. Insbesondere wird in manchen Ausführungsformen, bei denen das Atemgas auch im Beatmungsgerät 2 in einem Kreislauf geführt wird, durch das Beatmungsgerät 2 Sauerstoff in das Atemgas nachgeführt.

In manchen Ausführungsformen verfügt das Beatmungsgerät 2 über einen Atemgasanfeuchter und/oder eine Atemgasheizung und/oder das Beatmungsgerät 2 ist mit solchen Einrichtungen verbunden.

In manchen Ausführungsformen ist das Beatmungsgerät 2 als Anästhesiegerät ausgeführt, wobei dem Atemgas zusätzlich ein Anästhesiegas zugeführt wird. Insbesondere dann, wenn das Beatmungsgerät 2 als Anästhesiegerät ausgeführt ist, strömt das Atemgas aus dem Gaskreislauf 1a zurück in das Beatmungsgerät 2 und wird innerhalb des Beatmungsgerätes 2 zumindest teilweise in einem Kreislauf geführt. Während der Inspirations- und Exspirationsphasen findet dann zumindest teilweise ein Gasaustausch zwischen dem Gaskreislauf 1a des Lungensimulators 1 und dem Kreislauf des als Anästhesiegeräte ausgeführten Beatmungsgerätes 2 statt.

In manchen Ausführungsformen kann das Beatmungsgerät 2 auch eine Vorrichtung zur manuellen Beatmung sein, beispielsweise ein Beatmungsbeutel.

Das Beatmungsgerät 2 ist beispielhaft über ein Y-Stück (bzw. T-Stück, allgemein ein Verbindungsstück mit 3 Gaswegen) mit dem Gaskreislauf 1a des Lungensimulators 1 verbunden. In manchen Ausführungsformen sind zwischen dem Gaskreislauf 1a und dem Beatmungsgerät 2 Sensoren angeordnet, beispielsweise um Fluss, Druck, CO2-Konzentration, O2-Konzentration, Temperatur und/oder Feuchtigkeit des Atemgases zu messen. In manchen Ausführungsformen umfasst das Y-Stück, über welches der Gaskreislauf 1a mit dem Beatmungsgerät 2 verbunden ist beziehungsweise von dem eine Schlauchverbindung zum Beatmungsgerät 2 abgeht, zumindest ein Ventil, welches sich je nach Atemphase (Inspiration, Exspiration) öffnet und/oder schließt. In manchen Ausführungsformen ist eine Patientenschnittstelle, zum Beispiel eine Maske, zwischen Gaskreislauf 1a und Beatmungsgerät 2 angeordnet.

Beispielhaft kann der Verbindungspunkt, also zum Beispiel das Y-Stück, zwischen Gaskreislauf 1a und Beatmungsgerät 2 als Start- und Endpunkt des Gaskreislaufs 1a betrachtet werden. Von dem Verbindungpunkt strömt das Atemgas zunächst durch den Gastauscher 101, dann durch den CO2-Beimischer 102 und schließlich durch bzw. in und aus der Testlunge 103.

Die Vorrichtung 11 zur Einstellung der O2-Konzentration im Atemgas des Gaskreislaufs 1a ist beispielhaft als Gastauscher 101 ausgeführt. Der Gastauscher 101 ist beispielhaft so eingerichtet und ausgebildet, dass die O2-Konzentration in dem Atemgas, welches durch den Gaskreislauf 1a strömt, eingestellt werden kann. Beispielhaft wird durch den Gastauscher 101 eine Aufnahme von Sauerstoff aus dem Atemgas in einer Lunge simuliert, die O2-Konzentration in dem Atemgas des Gaskreislaufs 1a wird also verringert. Beispielhaft wird dazu Sauerstoff (O2) aus dem Atemgas entfernt. In manchen Ausführungsformen wird beispielsweise Sauerstoff über eine Membran aus dem Atemgas entfernt. Dazu wird beispielsweise ein Spülgas mit einer geringeren O2-Konzentration als das Atemgas auf einer Seite der Membran entlang geleitet und das Atemgas auf der anderen Seite der Membran. Ist die Membran zumindest für O2-Moleküle permeabel, so können O2-Moleküle aus dem Atemgas in das Spülgas diffundieren wodurch die O2-Konzentration im Atemgas sinkt.

In manchen Ausführungsformen ist der Gastauscher 101 so eingerichtet und ausgebildet, dass das Atemgas des Gaskreislaufs 1a mit einem Gas oder Gasgemisch gemischt wird, sodass die O2-Konzentration im Atemgas verdünnt wird. Ein solches Gas oder Gasgemisch kann beispielsweise Luft und/oder Stickstoff (N2) sein, welches gegebenenfalls mit Sauerstoff gemischt wird. Soll die O2-Konzentration im Atemgas verringert werden, so sollte das Gas beziehungsweise Gasgemisch, welches mit dem Atemgas gemischt wird, eine geringere O2-Konzentration aufweisen als das Atemgas. Um die Flussrate im Gaskreislauf 1a durch die Zugabe des Gases/Gasgemisches zum Atemgas gegenüber der durch das Beatmungsgerät 2 vorgegebenen Flussrate nicht zu erhöhen, kann beispielsweise ein Teil des verdünnten Atemgases wieder aus dem Gaskreislauf 1a ausgeleitet werden.

In manchen Ausführungsformen kann der Gastauscher 101 so eingestellt werden, dass je nach Bedarf die O2-Konzentration des Atemgases erhöht oder verringert werden kann.

In Flussrichtung nach der Vorrichtung 11 zur Einstellung der O2-Konzentration ist beispielhaft die Vorrichtung 12 zur Einstellung der CO2-Konzentration angeordnet. Beispielsweise ist die Vorrichtung 12 als CO2-Beimischer 102 ausgeführt. Über den CO2-Beimischer 102 wird beispielsweise die Abgabe von CO2 aus der Lunge in das Atemgas simuliert. Dazu wird die CO2-Konentration des Atemgases in dem Gaskreislauf 1a erhöht, beispielsweise durch zumindest zeitweise Zugabe von CO2 über den CO2-Beimischer 102. In manchen Ausführungsformen wird dabei die Zugabe von CO2 bereits bei der Einstellung der O2-Konzentration im Gastauscher 101 berücksichtigt, da eine zusätzliche Beimischung von CO2 zu dem Atemgas die O2-Konzentration verdünnt.

In manchen Ausführungsformen ist der CO2-Beimischer 102 so eingerichtet, dass über ein (regelbares) Ventil CO2 zumindest zeitweise in den Gaskreislauf 1a geleitet wird.

In manchen Ausführungsformen kann auch der CO2-Beimischer 102 wie der Gastauscher 101 ausgeführt sein, sodass über eine Membran CO2-Moleküle in und/oder aus dem Atemgas diffundieren können. Im manchen Ausführungsformen sind die Vorrichtung 11 zur Einstellung der O2-Konzentration und die Vorrichtung 12 zur Einstellung der CO2-Konzentration zusammen als ein Gastauscher 101 ausgeführt. Beispielsweise wird in dem Gastauscher 101 über eine Spülgas die O2- und CO2-Konzentration eingestellt und so die Aufnahme von O2 und die Abgabe von CO2 durch die Lunge simuliert. Beispielsweise weist das Spülgas dazu eine geringere O2-Konzentration und eine höhere CO2-Konzentration als das Atemgas auf.

In Flussrichtung nach der Vorrichtung 12 zur Einstellung der CO2-Konzentration ist die Vorrichtung 13 zur Simulation der mechanischen Lungenbewegung angeordnet. Beispielhaft ist die Vorrichtung 13 als Testlunge 103 ausgebildet. Die Testlunge 103 ist beispielsweise über ein Y-Stück, optional auch als steuerbares Ventil ausgeführt, in den Gaskreislauf 1a integriert, sodass Atemgas über die gleiche Leitung in und aus der Testlunge 103 strömen kann.

Im Allgemeinen ist die Testlunge 103 so ausgebildet und eingerichtet, dass ein Atemfluss während der Inspiration und der Exspiration simuliert wird. Während der Inspiration strömt dazu Atemgas in die Testlunge 103, beispielsweise bis ein gewisses Gasvolumen in der Testlunge 103 erreicht ist, und während der Exspiration strömt Atemgas aus der Testlunge 103 aus.

Die Testlunge 103 simuliert beispielsweise die Bewegung der Lunge während der Atmung. In manchen Ausführungsformen wird durch die Testlunge 103 aktiv die Lungenbewegung während der Atmung simuliert. Beispielsweise ist die Testlunge 103 dazu als Balg ausgeführt, welcher je nach Atemphase mechanisch expandiert und/oder komprimiert wird. Wird der Balg expandiert, so entsteht innerhalb der Testlunge 103 ein Unterdruck, welcher Atemgas aus dem Gaskreislauf 1a in die Testlunge 103 strömen lässt. So wird beispielsweise eine Inspiration simuliert.

Beispielsweise kann die Testlunge 103 so ausgeführt sein, dass die Expansion (also die Inspiration) nach einem gewissen Volumen, welches in die Testlunge 103 strömt, und/oder einer gewissen Zeit, in der Atemgas in die Testlunge 103 strömt, gestoppt wird und der Balg der Testlunge 103 wieder komprimiert wird. Dadurch entsteht beispielsweise ein Überdruck, welcher dazu führt, dass Atemgas aus der Testlunge 103 in den Gaskreislauf 1a strömen lässt. So wird beispielsweise die Exspiration simuliert. In manchen Ausführungsformen ist die Testlunge 103 so ausgeführt, dass die Komprimierung (also die Exspiration) nach einem gewissen Volumen, welches aus der Testlunge 103 strömt, und/oder einer gewissen Zeit, in welcher Atemgas aus der Testlunge 103 strömt, gestoppt wird. In manchen Ausführungsformen ist die Testlunge 103 so ausgebildet und eingerichtet, dass Inspiration und Exspiration im Wechsel simuliert werden.

Das Beatmungsgerät 2 ist beispielhaft dazu ausgebildet, die Bewegungen der Testlunge 103 als Inspiration und/oder Exspiration zu erkennen, beispielsweise über Druck- und/oder Flussänderungen, und entsprechend das durch das Beatmungsgerät 2 vorgegebene Druck- und/oder Flussniveau zu ändern (Wechsel zwischen Inspirations- und Exspirationsunterstützung).

In manchen Ausführungsformen kann vorgesehen sein, dass der Lungensimulator 1 auch ohne angeschlossenes Beatmungsgerät 2 betrieben werden kann. Das Atemgas im Gaskreislauf 1a wird dann beispielsweise hauptsächlich durch die simulierten Lungenbewegungen durch beispielsweise die Testlunge 103 bzw. die Vorrichtung 13 zur Simulation der mechanischen Lungenbewegung bewirkt.

In manchen Ausführungsformen des Lungensimulators 1 ist die Testlunge 103 passiv ausgeführt. In einer passiven Ausführung wird die Atembewegung nur passiv durch die Testlunge 103 simuliert. Beispielsweise ist dazu das Beatmungsgerät 2 dazu eingerichtet zumindest ein Druckniveau für eine Inspiration vorzugeben und ein Druckniveau für die Exspiration vorzugeben, wobei das Druckniveau für die Inspiration höher liegt als das Druckniveau für die Exspiration. Während der Inspiration strömt Atemgas in den Gaskreislauf 1a und in die Testlunge 103, wodurch die Testlunge 103 beispielsweise expandiert wird. In manchen Ausführungsformen umfasst die Testlunge 103 einen Widerstand, welcher mit zunehmender Expansion zunimmt und/oder ein maximales Limit für eine Expansion darstellt. Nach einer gewissen Zeit, welche die Inspirationsphase darstellt, wird das maximale Expansionslimit erreicht und/oder der Widerstand der Testlunge erreicht eine Größe, welche dem Inspirationsdruck des Beatmungsgerätes 2 entspricht, wodurch die Testlunge nicht weiter expandieren kann. Beispielsweise erreicht der Fluss aus dem Beatmungsgerät 2 in den Gaskreislauf 1a einen Wert, welcher durch das Beatmungsgerät 2 als Umschaltpunkt in die Exspiration erkannt wird. An diesem Umschaltpunkt wechselt beispielsweise das Beatmungsgerät 2 auf das Druckniveau der Exspiration, welches unter dem Druckniveau der Inspiration liegt. Dadurch verringert sich auch der Druck in dem Gaskreislauf 1a, was ein Strömen von Atemgas aus der Testlunge 103 ermöglicht. Zu Beginn der Exspiration übt der Widerstand der Testlunge 103 beispielsweise einen Druck aus, welcher über dem Exspirationsdruck des Beatmungsgerät 2 liegt, wodurch Atemgas aus der Testlunge 103 strömt. Nach einer gewissen Zeit ist die Testlunge 103 soweit komprimiert, dass der Widerstand dem Exspirationsdruck des Beatmungsgerät 2 entspricht, es kann also kein weiteres Atemgas aus der Testlunge 103 ausströmen. Das Beatmungsgerät 2 erkennt beispielsweise an dem Flusswert, dass die Exspiration abgeschlossen ist und/oder zeitnah abgeschlossen ist und kann beispielsweise wieder auf die Inspiration umschalten.

In manchen Ausführungsformen ist die Testlunge 103 ein einfacher Balg, welcher einen eigenen, vom Volumen abhängigen Widerstand aufweist. In manchen Ausführungsformen weist die Testlunge 103 einen Balg (oder etwas Vergleichbares) auf, welcher in einer Vorrichtung gelagert ist, über die ein Widerstand gegen das Expandieren vorgegeben ist. In manchen Ausführungsformen ist der Widerstand der Testlunge 103 zumindest Volumenabhängig. In manchen Ausführungsformen lässt sich der Widerstand der Testlunge 103 entsprechend eines maximalen Lungenvolumens einstellen, sodass verschiedene Lungenvolumina simulieren lassen.

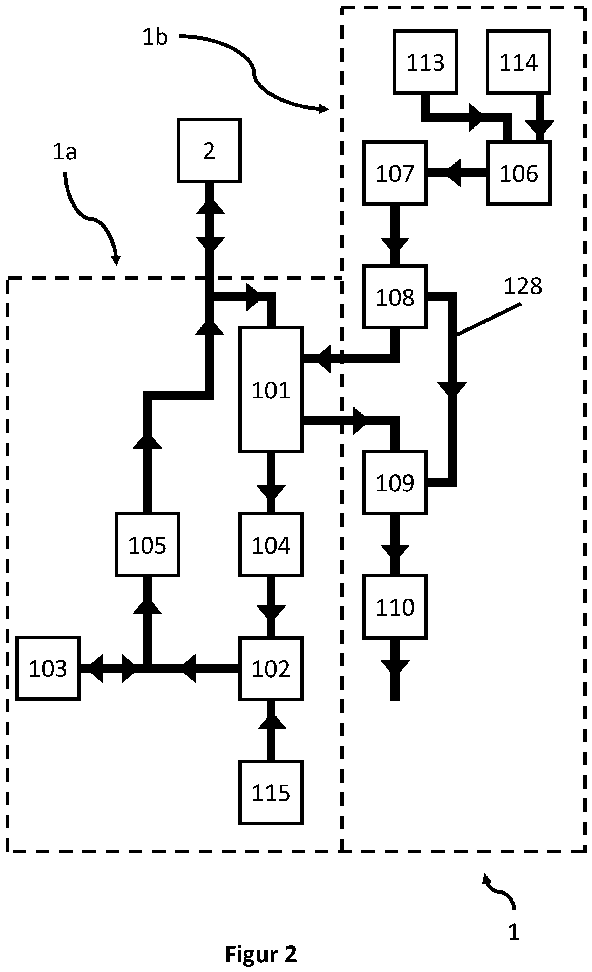

Eine weitere beispielhafte Ausführungsform des Lungensimulators 1 ist in Figur 2 schematisch dargestellt. Neben dem Gaskreislauf 1a umfasst der Lungensimulator 1 dabei auch noch einen Spülgaslauf 1b.

Das Beatmungsgerät 2 ist mit dem Gaskreislauf 1a des Lungensimulators 1 verbunden, sodass Atemgas von dem Beatmungsgerät 2 zumindest in den Gaskreislauf 1a strömen kann. In manchen Ausführungsformen sind Beatmungsgerät 2 und Lungensimulator 1 so verbunden, dass Atemgas zumindest zeitweise von dem Beatmungsgerät 2 in den Gaskreislauf 1a strömen kann und zumindest zeitweise aus dem Gaskreislauf 1a in Richtung Beatmungsgerät 2 strömen kann. In manchen Ausführungsformen kann Atemgas zumindest teilweise und zumindest zeitweise über ein Ventil und/oder einem Leckagesystem aus dem Gaskreislauf 1a entweichen. Insbesondere kann während der Exspirationsphase Atemgas aus dem Gaskreislauf 1a strömen. Beispielsweise kann das Beatmungsgerät 2 über ein Zweischlauchsystem mit dem Lungensimulator 1 verbunden sein.

In Flussrichtung nach der Verbindung zwischen Beatmungsgerät 2 und Gaskreislauf 1a ist der Gastauscher 101 angeordnet. In dem Gastauscher 101 wird zumindest die O2-Konzentration des Atemgases eingestellt. In der gezeigte, beispielhaften Ausführungsform wird mit dem Gastauscher 101 die Sauerstoffaufnahme aus dem Atemgas in der Lunge simuliert. Die O2-Konzentration des Atemgases wird also verringert. Der Gastauscher 101 ist beispielhaft so eingerichtet und ausgebildet, dass Sauerstoff aus dem Atemgas entfernt wird beziehungsweise aus dem Atemgas diffundiert. Beispielhaft findet die Einstellung der O2-Konzentration über ein Spülgas statt, welches durch einen Spülgaslauf 1b strömt. Das Spülgas und das Atemgas strömen dabei auf jeweils einer Seite einer Membran, welche zumindest für O2-Moleküle permeabel (durchlässig) ist. Weist das Spülgas eine geringere O2-Konzentration als das Atemgas auf, so diffundieren O2-Moleküle aus dem Atemgas durch die Membran in das Spülgas. Aus dem Atemgas wird also ein Teil des Sauerstoffs an der Membran des Gastauschers 101 herausgefiltert.

Als Spülgas wird zumindest ein Gas und/oder Gasgemisch verwendet, welches eine geringere O2-Konzentration aufweist als das Atemgas. In manchen Ausführungsformen kann Luft als Spülgas verwendet werden. In manchen Ausführungsformen wird Luft zusätzlich mit Sauerstoff und/oder Stickstoff versetzt. In manchen Ausführungsformen ist das Spülgas eine Mischung aus Sauerstoff und Stickstoff. Um das Spülgas bereitzustellen ist im Spülgaslauf 1b zumindest eine Gasquelle 113 angeordnet. Die Gasquelle 113 kann beispielsweise eine Gasdruckflasche und/oder ein Gebläse sein. In der beispielhaften Ausführungsform der Figur 2 sind im Spülgaslauf 1b zwei Gasquellen 113, 114 angeordnet. In manchen Ausführungsformen können auch drei und mehr Gasquellen angeordnet sein, beispielsweise um die Zusammensetzung des Spülgases mit weiteren Gasen zu versetzen. In manchen Ausführungsformen ist nach den Gasquellen 113, 114 jeweils zumindest ein Ventil angeordnet, beispielsweise um den Gasfluss anpassen zu können. In manchen Ausführungsformen ist zumindest eine der Gasquellen 113, 114 eine Druckgasquelle (Gasflasche, Druckgasleitung), wobei optional ein Druckminderer nach der Druckgasquelle angeordnet wird.

Die Gase aus den Gasquellen 113, 114 werden beispielhaft in einem Gasmischer 106 vermischt. Beispielhaft umfasst der Gasmischer 106 pro Gasquelle zumindest ein Ventil und eine Vorrichtung zur Messung des Gasflusses bzw. der Flussrate. Das Ventil ist beispielsweise so ausgebildet, dass über das Ventil der Gasfluss der jeweiligen Gasquelle 113,114 geregelt werden kann. In manchen Ausführungsformen ist das Ventil als elektrisch regelbares Ventil, beispielsweise als Proportionalventil ausgeführt. In manchen Ausführungsformen werden die Ventile über eine Steuereinheit geregelt. In manchen Ausführungsformen werden die Ventile über die Steuereinheit so geregelt, dass die Spülgaszusammensetzung automatisch so angepasst werden, dass über das Spülgas eine gewünschte O2-Konzentration im Atemgas eingestellt werden kann. Die Vorrichtung zur Messung des Gasflusses in dem Gasmischer 106 ist beispielsweise als Differenzdruckmesser ausgeführt. Es ist an dieser Stelle darauf hinzuweisen, dass auch beliebige andere Messanordnungen zur Bestimmung des Gasflusses bzw. der Flussrate verwendet werden können.

In dem Gasmischer 106 werden die Gase aus den Gasquellen 113, 114 vermischt, sodass ein Spülgas bereitgestellt wird, welches eine geringere O2-Konzentration aufweist als das Atemgas in dem Gaskreislauf 1a. Wird beispielsweise Luft mit einer O2-Konzentration von etwa 21% als Atemgas in dem Gaskreislauf 1a verwendet, so muss die O2-Konzentration des Spülgases unter 21% liegen, damit in dem Gastauscher 101 Sauerstoff von dem Atemgas in das Spülgas diffundiert. Beispielsweise kann dazu eine der Gasquellen 113, 114 eine Luftquelle sein und die andere Gasquelle 113, 114 eine Stickstoffquelle. In manchen Ausführungsformen ist es notwendig, dass der Unterschied der O2-Konzentration zwischen Atemgas und Spülgas nicht zu groß gewählt wird, beispielsweise um auch geringere Mengen an Sauerstoff aus dem Atemgas zu filtern.

In Flussrichtung nach dem Gasmischer 106 ist in dem Spülgaslauf 1b ein Sauerstoffsensor 107 angeordnet, welcher die O2-Konzentration in dem Spülgas vor dem Gastauscher misst.

Das Spülgas wird beispielhaft über ein Ventil 108 zu dem Gastauscher 101 geleitet. In manchen Ausführungsformen ist das Ventil 108 ein 3/2-Wege-Ventil. Durch Umschalten des Ventils 108 kann das Spülgas auch über den Bypass 128 geleitet werden, ohne, dass das Spülgas durch den Gastauscher 101 strömt. Der Bypass 128 dient beispielsweise zur Kalibrierung der Sauerstoffsensoren 107, 109.

In Flussrichtung nach dem Gastauscher 101 bzw. über den Bypass 128 nach dem Ventil 108 ist ein zweiter Sauerstoffsensor 109 angeordnet. Zusammen mit den Daten des ersten Sauerstoffsensors 107 kann so die Menge an Sauerstoff bestimmt werden, welche von dem Atemgas in das Spülgas diffundiert ist, also aus dem Atemgas entfernt/gefiltert wurde.

Beispielhaft ist nach dem zweiten Sauerstoffsensor 109 eine Vorrichtung zur Messung des Gasflusses bzw. der Flussrate angeordnet. In der gezeigten Ausführungsform ist hier beispielsweise ein Differenzdruckmesser 110 angeordnet. Der Spülgaslauf 1b endet beispielhaft mit einem Auslass in die Umgebungsluft. In manchen Ausführungsformen ist auch daran gedacht, dass Spülgas zu recyceln. Beispielsweise kann das Spülgas weiter mit Sauerstoff angereichert werden und zumindest teilweise als Atemgas für den Gaskreislauf 1a verwendet werden. In manchen Ausführungsformen kann das Spülgas auch im Kreislauf geführt werden, also wieder in den Spülgaslauf 1b geleitet werden. Gegebenenfalls muss hier wieder eine Anpassung der O2-Konzentration im Gasmischer 106 vorgenommen werden, damit eine weitere Sauerstoffaufnahme im Gastauscher 101 möglich ist.

In Flussrichtung nach dem Gastauscher 101 ist im Gaskreislauf 1a ein Ventil 104 angeordnet. Dieses Ventil 104 soll eine Strömung von Atemgas zurück, beispielsweise während der Exspirationsphase, in den Gastauscher 101 verhindern. Beispielhaft ist das Ventil 104 als Rückschlagventil ausgeführt, wobei die Strömung nur aus Richtung des Gastauschers 101 möglich ist. In manchen Ausführungsformen ist das Ventil 104 ein magnetisches und/oder elektrisch gesteuertes Ventil, welches während der Inspirationsphase geöffnet ist und während der Exspirationsphasen geschlossen ist. Beispielsweise kann das Ventil 104 dazu über eine Steuereinheit gesteuert werden, welche vom Beatmungsgerät 2 und/oder der Testlunge 103 Daten erhält, ob eine Exspirationsphase oder eine Inspirationsphase vorliegt.

In Flussrichtung nach dem Ventil 104 ist der CO2-Beimischer 102 angeordnet. Über den CO2-Beimischer wird die CO2-Konzentration des Atemgases eingestellt. Beispielhaft wird über den CO2-Beimischer 102 die Abgabe von CO2 in einer Lunge an das Atemgas simuliert. Entsprechend wird durch den CO2-Beimischer 102 die CO2-Konzentration im Atemgas erhöht. Beispielhaft wird dazu durch den CO2-Beimischer 102 aus einer CO2-Quelle CO2 in das Atemgas geleitet. Beispielhaft umfasst der CO2-Beimischer zumindest ein regelbares Ventil, über welches die Menge an CO2, welche in das Atemgas geleitet wird, geregelt wird. In manchen Ausführungsformen umfasst der CO2-Beimischer 102 eine Mischkammer zur besseren Durchmischung von Atemgas und CO2. Beispielhaft ist das Ventil, über welches der CO2-Beimischer 102 das CO2 in das Atemgas leitet ein Proportionalventil.

In manchen Ausführungsformen ist der CO2-Beimischer 102 so eingerichtet und ausgebildet, dass nur während der Inspirationsphase CO2 in das Atemgas gemischt wird. Beispielsweise wird dazu das Ventil des CO2-Beimischers 102 in Abhängigkeit von den simulierten und/oder durch das Beatmungsgerät 2 vorgegebenen Atemphasen (Inspiration, Exspiration) gesteuert.

In Flussrichtung nach dem CO2-Beimischer 102 ist beispielsweise über ein Y-Stück die Testlunge 103 mit dem Gaskreislauf 1a verbunden. Die Testlunge 103 (im Detail zu Figur 1 beschrieben) beispielhaft so eingerichtet, dass der Atemgasfluss durch die Lungenbewegung (Inspiration und Exspiration) simuliert wird. Während der Inspiration kann ein bestimmtes, in manchen Ausführungsformen einstellbares, Atemgasvolumen in die Testlunge 103 strömen. In manchen Ausführungsformen wird durch das Beatmungsgerät 2 nach einer gewissen, einstellbaren Inspirationszeit auf eine Exspiration umgestellt, wobei ein Atemgasvolumen aus der Testlunge 103 strömen kann bzw. ausströmt. In manchen Ausführungsformen erkennt das Beatmungsgerät 2 anhand des Gasflusses, dass eine Umstellung zwischen Inspiration und Exspiration erfolgen soll.

In Flussrichtung nach der Testlunge 103 aber vor der Verbindung zwischen Beatmungsgerät 2 und Gaskreislauf 1a ist beispielhaft ein Ventil 105 angeordnet. Das Ventil 105 verhindert, dass Atemgas während der Inspirationsphase entgegen der Flussrichtung strömen kann. Es wird als eine Strömung des Atemgases während der Inspiration in Flussrichtung von Beatmungsgerät 2 in Richtung Gastauscher 101 sichergestellt. Beispielsweise ist das Ventil 105 als Rückschlagventil ausgeführt, welches nur Flussrichtung öffnet. In manchen Ausführungsformen ist das Ventil 105 ein magnetisches und/oder elektrisch gesteuertes Ventil, welches während der Exspirationsphase geöffnet ist und während der Inspirationsphase geschlossen ist. Beispielsweise kann das Ventil 105 dazu über eine Steuereinheit gesteuert werden, welche vom Beatmungsgerät 2 und/oder der Testlunge 103 Daten erhält, ob eine Exspirationsphase oder eine Inspirationsphase vorliegt.

In manchen Ausführungsformen des Lungensimulators 1 sind zumindest im Gaskreislauf 1a Sensoren zur Bestimmung von Flussraten, Atemgasdrücken und Gaskonzentration (zumindest O2- und/oder CO2-Konzentration im Atemgas) angeordnet. Beispielsweise befindet sich vor und/oder dem Gastauscher 101 ein O2-Sensor zur Bestimmung der O2-Konzentration des Atemgases vor und/oder nach dem Gastauscher 101. Beispielsweise können die Werte genutzt werden um die Zusammensetzung des Spülgases im Spülgaslauf 1b anzupassen und somit auch die gefilterte Menge Sauerstoff aus dem Atemgas zu kontrollieren.

Vor und/oder nach dem CO2-Beimischer 102 kann in manchen Ausführungsformen ein CO2-Sensor im Gaskreislauf 1a angeordnet sein. Beispielsweise kann darüber die in das Atemgas eingemischte Menge an CO2 bestimmt werden. Beispielsweise lässt sich der CO2-Beimischer 102 über die von dem/den CO2-Sensor(en) bestimmten CO2-Konzentrationen kontrollieren.

Die Daten von den optionalen Sensoren im Gaskreislauf 1a können beispielsweise auch mit Daten und Werten, welche durch das Beatmungsgerät 2 bestimmt werden, verglichen werden. So kann zum Beispiel eine korrekte Funktion des Beatmungsgerätes 2 zusätzlich sichergestellt werden.

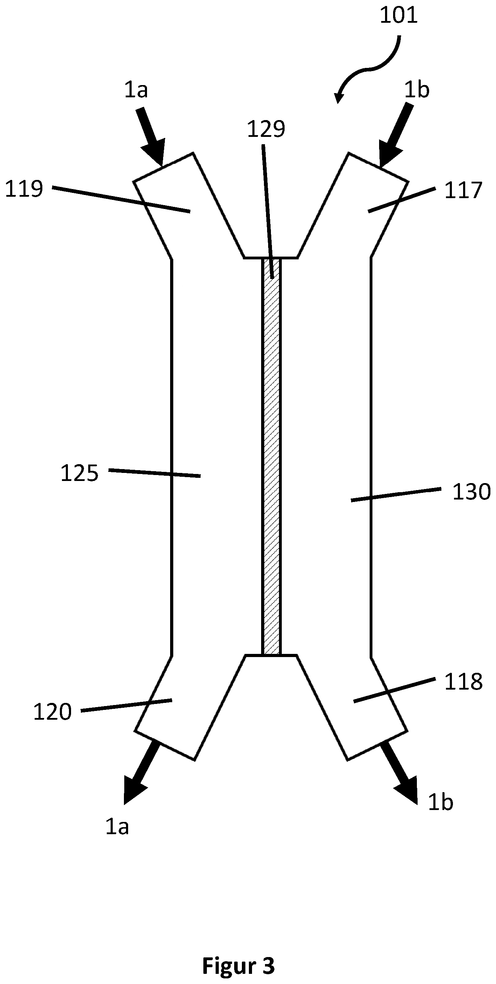

In Figur 3 ist eine beispielhafte Ausführungsform eines Gastauschers 101 schematisch dargestellt. Der Gastauscher 101 verfügt dabei über zumindest zwei Gasräume 125, 130, welche durch eine Membran 129 im Wesentlichen voneinander getrennt sind. Die Membran 129 ist beispielsweise für bestimmte Moleküle permeabel, zumindest aber für O2-Moleküle. In manchen Ausführungsformen ist die Membran zumindest auch für N2- und CO2-Moleküle permeabel. Über einen Anschluss 119 wird beispielhaft Atemgas aus dem Gaskreislauf 1a in einen Gasraum 125 und zumindest teilweise entlang der Membran 129 geleitet. Über einen Anschluss 117 wird beispielhaft das Spülgas aus dem Spülgaslauf 1b in den Gasraum 130 und zumindest teilweise entlang der Membran 129 geleitet.

Die Membran 129 ist zumindest für O2-Moleküle permeabel, sodass Sauerstoff von einem Gasgemisch mit höherer O2-Konzentration durch die Membran zu einem Gasgemisch mit geringerer O2-Konzentration diffundieren kann. Beispielsweise ist das Spülgas so eingestellt, dass die O2-Konzentration unterhalb der O2-Konzentration des Atemgases des Gaskreislaufes 1a liegt. Infolgedessen diffundiert Sauerstoff durch die Membran von dem Atemgas in das Spülgas, Sauerstoff wird also aus dem Atemgas entfernt bzw. gefiltert. Dadurch verringert sich die O2-Konzentration in dem Atemgas.

Beispielsweise ist die Membran aus Polymethylpenten und/oder Polypropylen gefertigt. Bevorzugt wird Polymethylpenten eingesetzt.

Über den Anschluss 120 wird das Atemgas wieder in den Gaskreislauf 1a geleitet und über den Anschluss 118 wird das Spülgas in den Spülgaslauf 1b geleitet. In manchen Ausführungsformen strömt das Atemgas mit der gleichen Flussrichtung wie das Spülgas. In manchen Ausführungsformen kann das Spülgas aber entgegen der Strömungsrichtung des Atemgases entlang der Membran geleitet werden. Auch eine Strömungsrichtung quer zum Atemgas ist möglich.

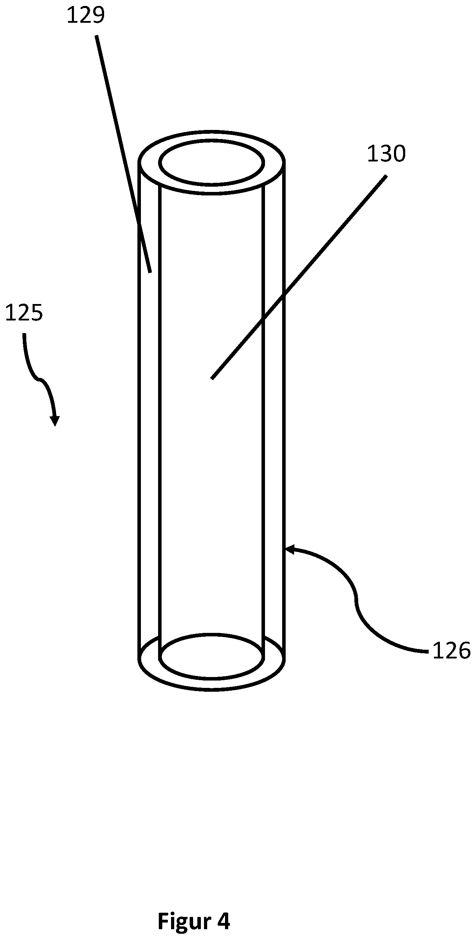

In manchen Ausführungsformen umgibt die Membran 129 den Gasraum 130, sodass eine Hohlfaser 126 ausgebildet wird, wie in Figur 4 schematisch dargestellt. Beispielsweise kann das Atemgas außen durch den Gasraum 125 an der Hohlfaser 126 vorbeiströmen, während das Spülgas beispielsweise innen durch den Gasraum 130 in der Hohlfaser 126 geleitet wird. Wie auch in der in Figur 3 beschriebenen Ausführungsform kann ein Gasaustausch zwischen dem Spülgas und dem Atemgas an bzw. durch die Membran 129 stattfinden. In manchen Ausführungsformen kann auch das Atemgas innen durch die Hohlfaser 126 geleitet werden, während das Spülgas außen an der Hohlfaser 126 vorbeigeleitet wird.

Als Hohlfasern 126 können beispielsweise Polymethylpenten- und/oder Polypropylen-Hohlfasern eingesetzt werden. Der innere Durchmesser der Hohlfasern liegt dabei in einem Bereich von 100 µm bis 500 µm, beispielsweise in einem Bereich um 200 µm (+/- 10%). Die Dicke der Membran liegt in einem Bereich von 50 µm bis 200 µm, bevorzugt zwischen 75 µm und 120µm, beispielsweise in einem Bereich um 90 µm (+/- 10%).

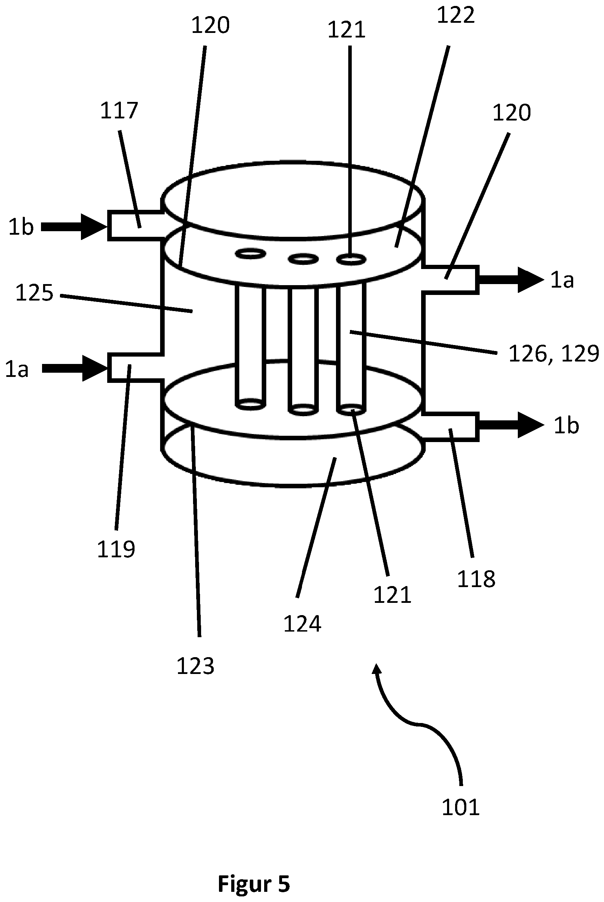

Ein beispielhafte Ausführungsform des Gastauschers 101 mit einer Vielzahl an Hohlfasern 126 ist schematisch in Figur 5 dargestellt. Dabei ist ein erster Gasraum 122 über eine Vielzahl an Hohlfasern 126 mit einem zweiten Gasraum 124 verbunden. Die Hohlfasern 126 verlaufen beispielsweise durch den Gasraum 125. Der Gasraum 125 ist über Dichtungen 120, 123 gegenüber dem ersten Gasraum 122 und dem zweiten Gasraum 124 abgedichtet. Durch den Gasraum 125 wird beispielsweise das Atemgas aus dem Gaskreislauf 1a geleitet, wobei das Atemgas an den Hohlfasern 126 zumindest teilweise vorbeiströmt. Beispielhaft wird das Spülgas über den Anschluss 117 in den ersten Gasraum 122 geleitet. Über Öffnungen 121 kann das Spülgas durch den Gasraum 130 innerhalb der Hohlfasern 126 in den zweiten Gasraum 124 strömen.

Entlang der Hohlfasern 126 kann es zu einem Gasaustausch zwischen dem Atemgas und dem Spülgas kommen. Beispielsweise kann Sauerstoff aus dem Atemgas mit einer höheren O2-Konzentration in das Spülgas mit einer geringeren O2-Konzentration diffundieren. Über eine sorgfältige Einstellung der Spülgaszusammensetzung und Flussrate des Spülgases kann gezielt die O2-Konzentration des Atemgases eingestellt werden. Beispielsweise kann so eingestellt werden, dass eine bestimmte Menge Sauerstoff aus dem Atemgas entfernt bzw. gefiltert wird, um die Sauerstoffaufnahme aus dem Atemgas in einer Lunge zu simulieren.

Beispielsweise strömt das Atemgas quer zur Strömungsrichtung des Spülgases. In manchen Ausführungsformen können die Anschlüsse 117, 118, 119, 120 so angeordnet sein, dass das Atemgas eine im Wesentlichen mit dem Spülgas übereinstimmende Strömungsrichtung aufweist und/oder eine im Wesentlichen dem Spülgas entgegengesetzte Strömungsrichtung aufweist. Insbesondere sind die Anschlüsse so angeordnet, dass im Wesentlichen der gesamte Gasraum 125 mit Atemgas durchströmt wird.

In manchen Ausführungsformen wird das Atemgas durch die Hohlfasern 126 vom ersten Gasraum 122 in den zweiten Gasraum 124 geleitet und das Spülgas wird durch den Gasraum 125 an den Hohlfasern 126 vorbeigeleitet.

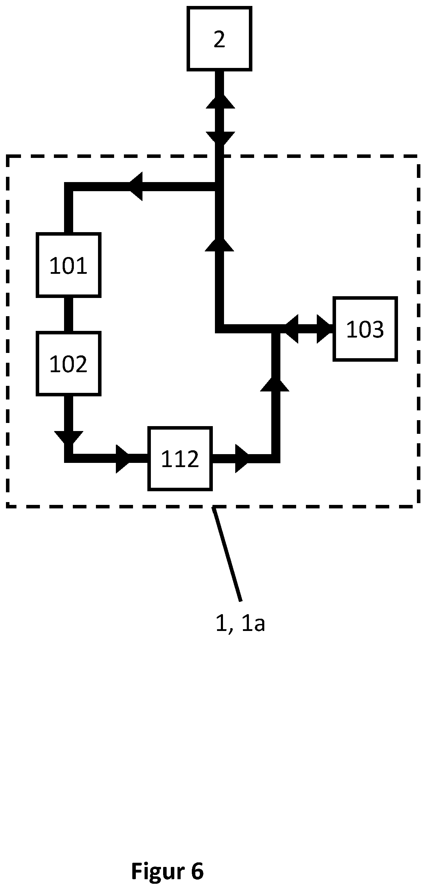

In einer weiteren beispielhaften Ausführungsform des Lungensimulators 1 ist im Gaskreislauf 1a ein Filter 112 angeordnet, wie in Figur 6 schematisch dargestellt. Beispielsweise ist der Filter 112 dazu angeordnet, eine Aufnahme von Anästhesiegasen aus dem Atemgas zumindest teilweise zu filtern. Der Filter 112 wird also insbesondere verwendet, wenn über das Beatmungsgerät 2 auch Anästhesiegase in den Gaskreislauf 1a strömen. Beispielsweise ist dazu das Beatmungsgerät 2 als Anästhesiegerät ausgeführt.

Im Wesentlichen entspricht die in Figur 6 dargestellte, beispielhafte Ausführungsform der zu Figur 1 beschriebenen, beispielhaften Ausführungsform, wobei zusätzlich ein Filter 112 im Gaskreislauf 1a angeordnet ist. Der Filter 112 ist beispielhaft dazu eingerichtet, Bestandteile aus dem Atemgas zu filtern, welche beispielsweise keine Bestandteile von reiner Luft sind. Insbesondere können über den Filter 112 zumindest Anästhesiegase und/oder Kohlenwasserstoffe und/oder halogenierte Kohlenwasserstoffe aus dem Atemgas gefiltert werden. In manchen Ausführungsformen werden zumindest CO2, O2 und N2 nicht oder in einem zu vernachlässigenden Anteil (<1%) durch den Filter 112 aus dem Atemgas gefiltert.

Beispielhaft ist der Filter 112 als Kohlefilter, etwa ein Aktivkohle-Filter, ausgeführt. Die Filterleistung kann beispielsweise durch die Oberfläche der Aktivkohle bestimmt werden. Auch die Verweilzeit des Atemgases im Filter 112 kann als Faktor genutzt werden, um die gefilterte Menge an Anästhesiegas einzustellen. Beispielsweise kann dazu die Größe (Breite, Länge) des Filters 112 angepasst werden. Auch andere Filterarten, wie zum Beispiel eine Polymermembran oder ein Zeolith-Filter sind als Filter 112 denkbar. In manchen Ausführungsformen ist auch eine Kombination von verschiedenen Filtermaterialien einsetzbar.

Der Filter 112 ist beispielhaft zwischen dem CO2-Beimischer 102 und der Testlunge 103 bzw. der Verbindung zur Testlunge 103 angeordnet.

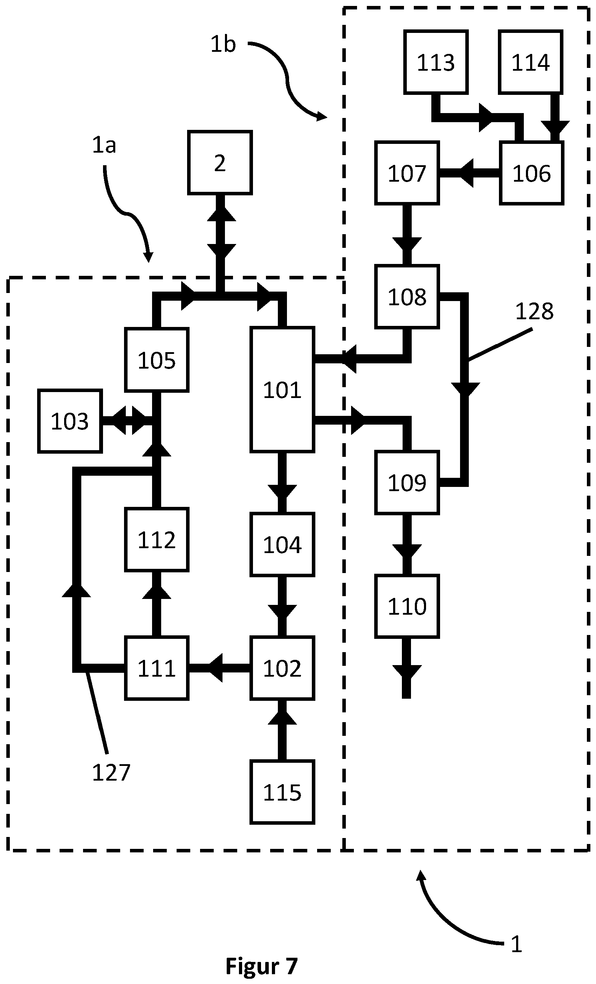

Figur 7 zeigt ein detaillierteres Schema der beispielhaften Ausführungsform des Lungensimulators 1 aus Figur 6. Der Aufbau stimmt dabei weitestgehend mit den in den Figuren 1 und 2 beschriebenen, beispielhaften Ausführungsformen überein, wobei zusätzlich ein Filter 112 in dem Gaskreislauf 1a angeordnet ist. Beispielhaft ist der Filter 112 zwischen dem CO2-Beimischer 102 und der Testlunge 103 bzw. der Verbindung zur Testlunge 103 angeordnet. In der dargestellten Ausführungsform ist zwischen dem Filter 112 und dem CO2-Beimischer 102 ein Bypass-Ventil 111 angeordnet. Über das Bypass-Ventil 111 kann zumindest ein Teil des Atemgas über den Bypass 127 an dem Filter 112 vorbei geleitet werden. Über die Atemgasmenge, welche über den Bypass 127 geleitet wird, kann beispielsweise auch eingestellt werden, welche Menge an Anästhesiegas aus dem Atemgas gefiltert wird. Der Bypass 127 wird dabei so geführt, dass über das Ventil 111 vor dem Filter 112 zumindest ein Teil des Atemgases an dem Filter vorbei geleitet wird und vor der Testlunge 103 wieder in den Gaskreislauf 1a strömt.

In manchen Ausführungsformen sind vor und/oder nach dem Filter 112 zusätzliche Sensoren angeordnet, welche zum Beispiel die Konzentration an Anästhesiegas im Atemgas messen können. In manchen Ausführungsformen ist auch vor der Verbindung mit der Testlunge 103 ein Sensor zur Messung der Anästhesiegaskonzentration im Atemgas angeordnet. In manchen Ausführungsformen wird das Bypass-Ventil 111 anhand der gemessenen Anästhesiegaskonzentration im Atemgas gesteuert.

Über das Bypass-Ventil 111 ist auch eine vollständige Umleitung des Atemgasstromes durch den Bypass 127 möglich, beispielsweise wenn der Lungensimulator 1 nicht mit einem Anästhesiegerät verwendet wird und kein Atemgas durch den Filter 112 gefiltert werden soll.

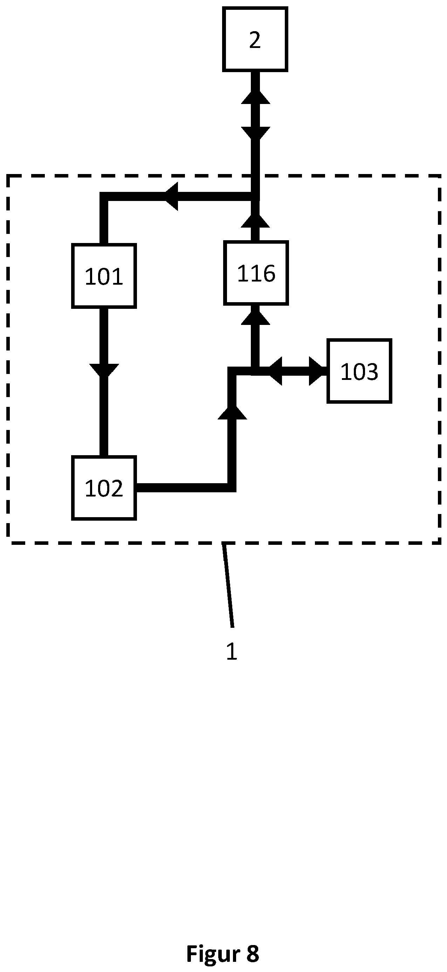

Ein weitere beispielhafte Ausführungsform des Lungensimulators 1 ist in Figur 8 schematisch dargestellt. Der Aufbau stimmt dabei weitestgehend mit den in den Figuren 1 und 2 beschriebenen, beispielhaften Ausführungsformen überein. Zusätzlich ist in der in Figur 8 dargestellten Ausführungsform ein Atemgasanfeuchter 116 im Gaskreislauf 1a angeordnet. Der Atemgasanfeuchter 116 simuliert zum Beispiel die Befeuchtung des Atemgases während der Atmung. Beispielhaft ist der Atemgasanfeuchter 116 in Flussrichtung zwischen der Testlunge 103 und dem Anschluss zum Beatmungsgerät 2 angeordnet. Der Atemgasanfeuchter 116 ist beispielsweise so eingerichtet, dass das Atemgas des Gaskreislaufs 1a angefeuchtet werden kann. Beispielsweise umfasst der Atemgasanfeuchter 116 dazu zumindest einen Flüssigkeitsbehälter, z.B. für Wasser. Der Gasstrom des Atemgases wird so durch den Atemgasanfeuchter 116 geleitet, dass der Atemgasstrom Feuchtigkeit, beispielsweise aus der Luft oberhalb des Flüssigkeitsspiegels, von der Flüssigkeit im Flüssigkeitsbehälter aufnimmt. In manchen Ausführungsformen wird der Atemgasstrom dazu über den Flüssigkeitsspiegel geleitet. In manchen Ausführungsformen umfasst der Atemgasanfeuchter 116 zumindest ein Heizelement, welches dazu eingerichtet ist, die Flüssigkeit zumindest teilweise zu erwärmen um die Luftfeuchtigkeit oberhalb des Flüssigkeitsspiegels zu erhöhen, beispielsweise um eine höhere Befeuchtung des Atemgases zu erreichen. In manchen Ausführungsformen ist zusätzlich ein Heizelement vorgesehen, welches das Atemgas zusätzlich erwärmt. Es ist zudem denkbar, dass vor und/oder nach dem Atemgasanfeuchter 116 ein Feuchtigkeitssensor angeordnet ist, welcher die Feuchtigkeit des Atemgases im Gaskreislauf 1a misst.

In manchen Ausführungsformen des Lungensimulators 1 ist die Anordnung des Atemgasanfeuchters 116 auch zusammen mit dem Filter 112 denkbar. In manchen Ausführungsformen ist der Atemgasanfeuchter 116 in Flussrichtung nach dem Filter 112 angeordnet, beispielsweise um eine ungewollte Befeuchtung des Filtermaterials im Filter 112 zu vermeiden. In manchen Ausführungsformen ist der Atemgasanfeuchter 116 in Flussrichtung unmittelbar vor dem Ventil 105 (siehe Figuren 2 und 7) angeordnet.

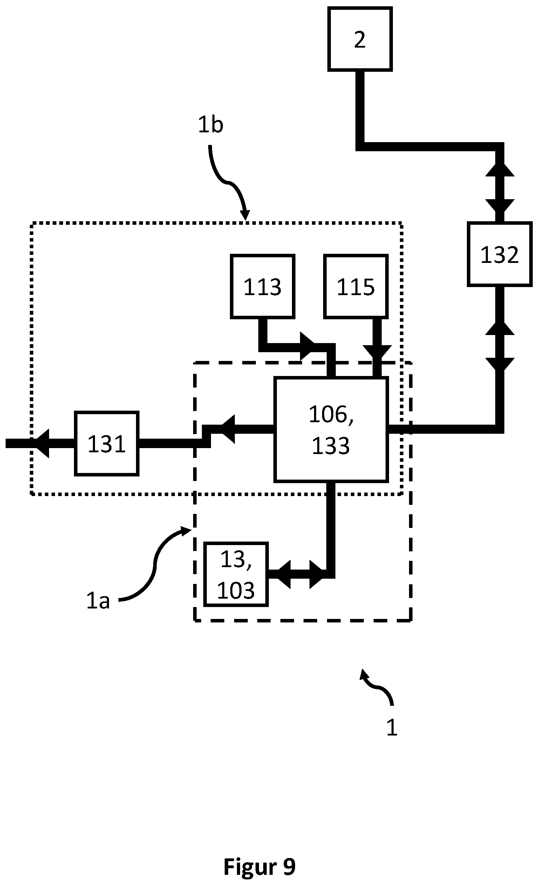

In Figur 9 ist eine weitere beispielhafte Ausführungsform des Lungensimulators 1 schematisch dargestellt. Zwischen dem Beatmungsgerät 2 und dem Lungensimulator 1 ist beispielhaft ein künstlicher Kopf 132 angeordnet. Die Verbindung zwischen Beatmungsgerät 2 und künstlichem Kopf 132 kann beispielsweise über ein Patienteninterface, etwa eine Maske, hergestellt werden. Um die Gaszusammensetzung im Bereich des künstlichen Kopfes 132 bzw. der Maske messen zu können, kann beispielsweise ein oder mehrere Gassensoren, etwa ein CO2-Sensor und/oder ein O2-Sensor, im künstlichen Kopf angeordnet sein. An verschiedenen Punkten des Lungensimulators kann zudem vorgesehen sein, dass Flusssensoren angeordnet sind. Beispielsweise kann vorgesehen sein, dass zumindest im künstlichen Kopf 132 ein Flusssensor angeordnet ist um die Flüsse und/oder Volumina zu bestimmen, welche während der simulierten Inspiration vom Beatmungsgerät 2 in den Lungensimulator 1 strömen und während der Exspiration den Fluss und/oder das Volumen bestimmt, welches aus dem Lungensimulator 1 durch den künstlichen Kopf 132 strömt.

Das Beatmungsgerät 2 fördert dabei zumindest während einer simulierten Inspiration Atemgas in die Richtung des Lungensimulators 1. Dabei kann vorgesehen sein, dass die Inspiration aktiv durch die Testlunge 103 simuliert wird oder eine Beatmung durch das Beatmungsgerät 2 ist oder eine Mischform aus beiden ist. Zur Simulation der aktiven Atmung durch den Lungensimulator 1 kann in manchen Ausführungsformen die Testlunge 103 auch eine Pumpe umfassen oder eine andere Vorrichtung, welche die Atembewegung simuliert. Das vom Beatmungsgerät 2 geförderte Atemgas gelangt in dem Lungensimulator 1 in den Gaskreislauf 1a, welcher zumindest durch den Gasmischer 106, hier als Mischkammer 133 ausgeführt, und die Testlunge 103 führt. In der Mischkammer 133 trifft der Gaskreislauf 1a auch auf den Spülgaslauf 1b. Das Spülgas wird dabei durch zumindest zwei Gasquellen 113, 115 bereitgestellt. In der gezeigten Ausführungsform ist eine der Gasquellen 113, 115 eine CO2-Quelle 115. Um eine definierte Menge an Sauerstoff aus dem Atemgas zu entfernen wird ein Teil des Gases über die mit der Mischkammer 133 verbundene Pumpe 131 aus dem Gaskreislauf 1a entfernt. Die definierte Menge an Sauerstoff wird durch eine gleiche Menge an CO2 aus der CO2-Quelle 115 ersetzt, sodass nach der Mischung der Gase ein Atemgas entsteht, bei welchem der Verlust an Sauerstoffkonzentration durch einen entsprechenden Gewinn an CO2 Konzentration ausgeglichen wird. In manchen Ausführungsformen kann zusätzlich berücksichtigt werden, dass bei der Atmung in der Regel mehr Sauerstoff aufgenommen wird als CO2 abgegeben wird. Es kann also ergänzend vorgesehen sein, dass eine um einen entsprechenden Faktor abweichende Menge an CO2 nachgeführt wird im Vergleich zu der abgepumpten Menge an Sauerstoff. Bei der Atmung wird in der Regel zwischen 1/5 und 1/8, beispielsweise 1/6, weniger CO2 abgegeben als Sauerstoff aufgenommen. Da zusammen mit dem Sauerstoff auch Atemgas, etwa CO2 und N2, über die Pumpe 131 abgesaugt wird, ist es zudem auch nötig die Mengen an N2 und CO2 nachzuliefern, welche entfernt wurden um die Volumenbilanz auszugleichen. So wird sichergestellt, dass das während der Inspiration gelieferte Volumen auch dem Volumen der Exspiration entspricht. Für eine bessere Durchmischung der Gase in dem Gasmischer 106 kann optional auch eine Vorrichtung zur Konvektion, etwa ein Lüfter, vorgesehen sein. Die entfernten Mengen an Sauerstoff, CO2 und N2 werden beispielsweise zum einen über entsprechende Gassensoren, zumindest aber CO2- und Sauerstoffsensoren, sowie über eine Messung des über die Pumpe 131 abgesaugten Volumens bestimmt. Alternativ oder ergänzend kann das Volumen auch über eine bekannte Pumpenkennlinie und z.B. die vorgegebene Frequenz/Drehzahl bestimmt werden.

Die CO2- und Sauerstoffsensoren sind beispielsweise in der Mischkammer angeordnet. Alternativ oder ergänzend kann auch vorgesehen sein, dass die CO2- und/oder Sauerstoffsensoren mit der Mischkammer 133 verbunden sind, also außerhalb der Mischkammer 133 angeordnet sind. Dazu kann vorgesehen sein, dass ein Gasstrom erzeugt wird, sodass der Gaskreislauf 1a durch die entsprechenden Sensoren strömt.

Es kann beispielsweise vorgesehen sein, dass für jeden Atemzyklus zunächst der Sauerstoff durch die Pumpe 131 entfernt wird und anschließend das Volumen durch zuströmen von zusätzlichem CO2 und N2 aus den Gasquellen 113, 115 ausgeglichen wird. In manchen Ausführungsformen kann auch vorgesehen sein, dass über die Gasquellen 113, 115 zunächst eine Zielkonzentration von O2 und CO2 eingestellt wird und dann über die Pump 131 überschüssiges Volumen abgesaugt wird.

In manchen Ausführungsformen ist vorgesehen, dass immer die gleiche Menge (Anzahl an Molekülen) Sauerstoff entfernt wird und durch eine gleiche Menge CO2 ersetzt wird, welche einer Umsetzung im Körper von Sauerstoff zu CO2 entspricht. Dadurch kann ein gleichbleibender O2-Vebrauch simuliert werden und beispielweise Effekte wie CO2-Akkumulation, etwa durch Rückatmung, geprüft werden. Insbesondere kann so möglich sein den Effekt der Maske auf die Atemgaslieferung zu überprüfen.

Die Verbindung zwischen dem künstlichen Kopf 132 und dem Lungensimulator 1 bzw. der Mischkammer 133 kann so gewählt sein, dass sie in Länge, Durchmesser und/oder Volumen einer Luftröhre entspricht. Zudem kann vorgesehen sein, dass die Verbindung zwischen künstlichem Kopf 132 und der Mischkammer 133 in den Maßen variabel ist um beispielsweise verschiedene Körpergrößen nachstellen zu können. Gleiches kann auch für die Mischkammer 133 und/oder die Testlunge 103 gelten. Beispielsweise kann über die Größe der Mischkammer 133 und/oder der Testlunge 103 auch ein bestimmtes Lungenvolumen simuliert werden.

In manchen Ausführungsformen des Lungensimulators 1 kann auch vorgesehen sein, dass kein Beatmungsgerät 2 angeschlossen wird. Die Gasströmung innerhalb des Gaskreislaufs 1a und/oder in/aus den Gaskreislauf 1a, beispielsweise durch den künstlichen Kopf 132, wird dabei beispielsweise durch die Vorrichtung 13 zur Simulation der mechanischen Lungenbewegung, beispielsweise eine Testlunge 103, realisiert. So kann beispielsweise die natürliche Atmung eines Lebewesens ohne zusätzliche Beatmung simuliert werden.

### Bezugszeichenliste

- 1: Lungensimulator
- 1a: Gaskreislauf
- 1b: Spülgaslauf
- 2: Beatmungsgerät
- 11: Vorrichtung (zur Einstellung der O2-Konzentration)
- 12: Vorrichtung (zur Einstellung der CO2-Konzentration)
- 13: Vorrichtung (zur Simulation der mechanischen Lungenbewegung)
- 101: Gastauscher
- 102: CO2-Beimischer
- 103: Testlunge
- 104: Ventil
- 105: Ventil
- 106: Gasmischer
- 107: Sauerstoffsensor
- 108: Ventil
- 109: Sauerstoffsensor
- 110: Differenzdruckmesser
- 111: Ventil
- 112: Filter
- 113: Gasquelle
- 114: Gasquelle
- 115: CO2 Quelle
- 116: Atemgasanfeuchter
- 117: Anschluss
- 118: Anschluss
- 119: Anschluss
- 120: Anschluss
- 121: Öffnung
- 122: Gasraum
- 123: Dichtung
- 124: Gasraum
- 125: Gasraum
- 126: Hohlfaser
- 127: Bypass
- 128: Bypass
- 129: Membran
- 130: Gasraum
- 131: Pumpe
- 132: Kunstkopf
- 133: Mischkammer

## Patentansprüche

1. Lungensimulator (1) zur teilweisen Simulation von Funktionen einer Lunge, umfassend zumindest einen Gaskreislauf (1a), wobei der Gaskreislauf (1a) mit einem Beatmungsgerät (2) verbunden ist, wobei das Beatmungsgerät (2) dazu eingerichtet ist, zumindest zeitweise ein Atemgas in und/oder aus den Gaskreislauf (1a) zu fördern, **dadurch gekennzeichnet, dass** der Lungensimulator (1)
a. zumindest eine Vorrichtung (11) zur Einstellung der O2-Konzentration des Atemgases in dem Gaskreislauf (1a),
b. zumindest eine Vorrichtung (12) zur Einstellung der CO2-Konzentration des Atemgases in dem Gaskreislauf (1a) und
c. zumindest eine Vorrichtung (13) zur Simulation der mechanischen Lungenbewegung
umfasst.

2. Lungensimulator (1) nach zumindest einem der vorhergehenden Ansprüche, **dadurch gekennzeichnet, dass** der Lungensimulator (1) einen Spülgaslauf (1b) umfasst, durch welchen ein Spülgas strömt und, dass die Vorrichtung (11) zur Einstellung der O2-Konzentration ein Gastauscher (101) ist, wobei der Gastauscher (101) dazu eingerichtet ist über das Spülgas des Spülgaslaufs (1b) die O2-Konzentration des Atemgases in dem Gaskreislauf (1a) einzustellen.

3. Lungensimulator (1) nach zumindest einem der vorhergehenden Ansprüche, **dadurch gekennzeichnet, dass** in dem Gastauscher (101) zumindest stellenweise und/oder zeitweise O2-Moleküle und/oder CO2-Moleküle und/oder N2-Moleküle vom Atemgas in das Spülgas übergehen und/oder vom Spülgas in das Atemgas übergehen, wobei der Gastauscher (101) zumindest eine Membran (129) umfasst und die Membran (129) zumindest für CO2 und/oder O2 und/oder N2 permeabel ist, sodass dass entlang der Membran (129) zumindest O2-Moleküle und/oder CO2-Moleküle und/oder N2-Moleküle vom Atemgas in das Spülgas übergehen und/oder vom Spülgas in das Atemgas übergehen.

4. Lungensimulator (1) nach zumindest einem der vorhergehenden Ansprüche, **dadurch gekennzeichnet, dass** der Gastauscher (101) zumindest zwei Gasräume (125, 130) umfasst, wobei die beiden Gasräume (125, 130) zumindest durch die Membran (129) voneinander getrennt sind und der Gastauscher (101) so eingerichtet und ausgebildet ist, dass ein Atemgas auf der einen Seite an der mindestens einen Membran (129) und ein Spülgas auf der anderen Seite der mindestens einen Membran (129) zumindest stellenweise entlang strömt, wobei die zumindest eine Membran (129) als zumindest eine Hohlfaser (126) ausgebildet ist, wobei die zumindest eine Hohlfaser (126) einen der zwei Gasräume (125, 130) umschließt.

5. Lungensimulator (1) nach zumindest einem der vorhergehenden Ansprüche, **dadurch gekennzeichnet, dass** der Gastauscher (101) zumindest vier Gasräume (122, 124, 125, 130) umfasst, wobei ein erster Gasraum (122) durch zumindest eine Hohlfaser (126) gasleitend mit einem zweiten Gasraum (124) verbunden ist und die zumindest eine Hohlfaser (126) durch den dritten Gasraum (125) läuft und wobei die zumindest eine Hohlfaser (126) so eingerichtet und ausgebildet ist, dass zumindest teilweise ein Gasaustausch zwischen dem Spülgas in der Hohlfaser (126) und dem die Hohlfaser (126) umströmenden Atemgas stattfindet.

6. Lungensimulator (1) nach zumindest einem der vorhergehenden Ansprüche, **dadurch gekennzeichnet, dass** der Spülgaslauf (1b) zumindest eine Gasquelle (113, 114, 115) umfasst, aus welcher ein Gas in den Spülgaslauf (1b) geleitet wird und wobei das Spülgas aus zumindest zwei Gasen besteht, welche aus zumindest zwei Gasquellen (113, 114, 115) in den Spülgaslauf (1b) geleitet werden und in einem Gasmischer (106) gemischt werden.

7. Lungensimulator (1) nach zumindest einem der vorhergehenden Ansprüche, **dadurch gekennzeichnet, dass** eine der zumindest zwei Gasquellen (113, 114, 115) eine Sauerstoffquelle und/oder eine CO2-Quelle ist und aus einer der zumindest zwei Gasquellen (113, 114, 115) Luft und/oder Stickstoff in den Spülgaslauf (1b) geleitet wird.

8. Lungensimulator (1) nach zumindest einem der vorhergehenden Ansprüche, **dadurch gekennzeichnet, dass** im Spülgaslauf (1b) zumindest ein Ventil (108) angeordnet ist, wobei das zumindest eine Ventil (108) ein 3/2-Wege-Ventil ist und das Ventil (108) zwischen einem ersten Sauerstoffsensor (107), dem Gastauscher (101) und einem zweiten Sauerstoffsensor (109) angeordnet ist, wobei der zweite Sauerstoffsensor (109) mit dem Ventil (108) und dem Gastauscher (101) gasleitend verbunden ist und in dem Spülgaslauf (1b) zumindest ein Differenzdruckmesser (110) in Flussrichtung nach dem Gastauscher (101) angeordnet ist.

9. Lungensimulator (1) nach zumindest einem der vorhergehenden Ansprüche, **dadurch gekennzeichnet, dass** die Vorrichtung (12) zur Einstellung der CO2-Konzentration des Atemgases in dem Gaskreislauf (1a) ein CO2-Beimischer (102) ist, wobei die Vorrichtung (12) zur Einstellung der CO2-Konzentration in Flussrichtung im Gaskreislauf (1a) nach der Vorrichtung (11) zur Einstellung der O2-Konzentration angeordnet ist und der CO2-Beimischer (102) mit einer CO2-Quelle (115) verbunden ist, wobei über den CO2-Beimischer CO2 in das Atemgas in dem Gaskreislauf (1a) eingeleitet wird und der CO2-Beimischer (102) zumindest ein Ventil umfasst, welches mit dem Gaskreislauf (1b) verbunden ist.

10. Lungensimulator (1) nach zumindest einem der vorhergehenden Ansprüche, **dadurch gekennzeichnet, dass** im Gaskreislauf (1a) die Vorrichtung (13) zur Simulation der mechanischen Lungenbewegung in Flussrichtung nach der Vorrichtung (12) zur Einstellung der CO2-Konzentration angeordnet ist und zwischen dem Gastauscher (101) und der Testlunge (103) die Verbindung des Beatmungsgerätes (2) mit dem Gaskreislauf (1a) angeordnet ist.

11. Lungensimulator (1) nach zumindest einem der vorhergehenden Ansprüche, **dadurch gekennzeichnet, dass** zwischen der Vorrichtung (11) zur Einstellung der O2-Konzentration und der Vorrichtung (13) zur Simulation der mechanischen Lungenbewegung zumindest ein Ventil (104) angeordnet ist und in Flussrichtung nach der Vorrichtung (13) zur Simulation der mechanischen Lungenbewegung zumindest ein Ventil (105) angeordnet ist, wobei das eine Ventil (104) zwischen dem Gastauscher (101) und dem CO2-Beimischer (102) angeordnet ist und das andere Ventil (105) zwischen der Testlunge (103) und der Verbindung des Beatmungsgerätes (2) mit dem Gaskreislauf (1a) angeordnet ist, wobei die Ventile (104, 105) Rückschlagventile und/oder steuerbare Ventile sind.

12. Lungensimulator (1) nach zumindest einem der vorhergehenden Ansprüche, **dadurch gekennzeichnet, dass** die Vorrichtung (12) zur Einstellung der CO2-Konzentration des Gases im Gaskreislauf (1a) ein Gastauscher (101) ist und die Vorrichtung (12) zur Einstellung der CO2-Konzentration und die Vorrichtung (11) zur Einstellung der O2-Konzentration in einem Gastauscher (101) kombiniert sind.

13. Lungensimulator (1) nach zumindest einem der vorhergehenden Ansprüche, **dadurch gekennzeichnet, dass** in dem Gaskreislauf (1a) zumindest eine Vorrichtung zur Simulation von Blockaden der Atemwege und/oder Apnoen und/oder Atemaussetzern angeordnet ist.

14. Lungensimulator (1) nach zumindest einem der vorhergehenden Ansprüche, **dadurch gekennzeichnet, dass** zwischen dem Lungensimulator (1) und dem Beatmungsgerät (2) ein künstlicher Kopf (132) mit Maske angeordnet ist und der Gaskreislauf (1a) zumindest teilweise durch den künstlichen Kopf (132) führt und der Gaskreislauf (1a) und der Spülgaslauf (1b) in einem Gasmischer (106) zusammentreffen, wobei der Gasmischer (106) als Mischkammer (133) ausgeführt ist und wobei eine Pumpe (131) mit der Mischkammer (133) verbunden ist, über welche zumindest eine definierte Menge an Sauerstoff aus der Mischkammer (133) entfernt wird und wobei die entfernte Menge Sauerstoff durch eine entsprechende Menge an CO2 aus einer Gasquelle (115) ersetzt wird.

15. Verfahren zur teilweisen Simulation von Funktionen einer Lunge, **dadurch gekennzeichnet, dass** in einem Lungensimulator (1) nach zumindest einem der vorhergehenden Ansprüche die O2-Konzentration und die CO2-Konzentration eines in einem Gaskreislauf (1a) geführten Atemgases eingestellt werden und die mechanischen Lungenbewegungen simuliert werden.
